# EUROPÄISCHE PATENTSCHRIFT

(11) **EP 1 680 176 B1**
(45) Veröffentlichungstag und Bekanntmachung des Hinweises auf die Patenterteilung: **23.08.2017**
(21) Anmeldenummer: 04790514.6
(22) Anmeldetag: 15.10.2004
(51) Int. Cl.: A61M 39/28, A61M 5/142

(54) **EINLEGEVORRICHTUNG**
INSERT DEVICE
DISPOSITIF D'INSERTION

(30) Priorität: 15.10.2003 DE 10348653
(43) Veröffentlichungstag der Anmeldung: 19.07.2006
(73) Patentinhaber: pfm medical tpm gmbh, 50996 Köln (DE)
(72) Erfinder: GOTTSCHALK, Andreas, 53913 Swisttal-Heimerzheim (DE)
(74) Vertreter: Ring & Weisbrodt
(86) Internationale Anmeldenummer: PCT/EP2004/011675
(87) Internationale Veröffentlichungsnummer: WO 2005/037349

(56) Entgegenhaltungen:
- WO-A-98/13080
- US-A- 4 586 691
- US-A- 4 689 043
- US-A- 5 437 642
- US-A- 5 946 738

## Beschreibung

Die Erfindung betrifft eine Einlegevorrichtung für eine Pumpvorrichtung, insbesondere eine Infusionspumpe, mit zumindest einer ersten Aussparung oder Ausnehmung zum Einlegen einer Leitung und mit zumindest einer Freiflussklemmeinrichtung.

Derartige Einlegevorrichtungen für Pumpeinrichtungen, die mit Freiflussklemmeinrichtungen versehen sind, sind im Stand der Technik bekannt. Eine Freiflussklemmeinrichtung dient zum Klemmen des in die Kassette eingelegten flexiblen Schlauchs dann, wenn die Kassette nicht in die Pumpvorrichtung eingelegt ist und dann, wenn die Verschlussklappe der Pumpvorrichtung geöffnet ist. Das Durchfließen von Medium durch den Schlauch wird dadurch unterbunden. Beispielsweise offenbart die EP 0 813 430 B1 eine mit einer Freiflussklemmeinrichtung versehene Kassette. In der Kassette ist ein Gehäuse der Freiflussklemmeinrichtung vorgesehen, in dem ein vertikal bewegliches, den Medienfluss durch den Schlauch stoppendes Element sowie eine Feder angeordnet sind. In dem den Fluss stoppenden Element ist eine Durchgangsöffnung vorgesehen, durch die der flexible Schlauch hindurchgeführt ist. Die Feder spannt das Stoppelement nach oben vor. Solange die Klappe der Pumpvorrichtung geschlossen ist, wird das den Fluss stoppende Element gegen die Federkraft niedergedrückt und das Medium kann durch den Schlauch strömen. Sobald die Klappe geöffnet wird oder die Kassette nicht innerhalb der Pumpvorrichtung angeordnet ist, bewegt die Federkraft das den Fluss stoppende Element nach oben, wodurch der in dieses eingefügte flexible Schlauch von der Unterseite her einseitig gegen die gegenüberliegende Wandung gedrückt und damit ein Flüssigkeitsstrom verhindert wird. Eine Arretierung der Freiflussklemmeinrichtung kann durch einen Stift erfolgen, der in eine Querbohrung des den Fluss stoppenden Elementes eingefügt wird. Dadurch wird das stoppende Element daran gehindert, durch die vorgespannte Feder nach oben gedrückt zu werden. Es wird dadurch jederzeit ein Freifluss möglich. Bei dieser Kassette erweist es sich als nachteilig, dass aufgrund der Federvorspannung und durch das sich in einem Gehäuse bewegende, den Durchfluss stoppende Element, durch das der flexible Schlauch hindurchgeführt ist, den Schlauch während des Abklemmvorganges verletzen kann, da er über die Gehäusekanten eingedrückt wird.

Eine andere Kassette bzw. Einlegevorrichtung für eine Pumpvorrichtung ist aus der EP 0 655 004 B1 und EP 0 847 769 B1 bekannt. Diese Druckschriften beschreiben ein mehrkanaliges Peritonealdialysesystem, wobei mehrere einzelne Beutel mit sterilen Peritonealdialyselösungen mit einem Peritonealdauerkatheter verbunden werden. Es ist eine Flüssigkeitsabsperreinrichtung vorgesehen, die dazu dient, jeglichen Flüssigkeitsdurchfluss durch die Kassette im Falle eines Stromausfalls oder eines sonstigen Fehlerzustandes abzusperren. Die Flüssigkeitsabsperreinrichtung weist einen beweglichen Verschlusskörper auf, der hinter einem Druckplattenrahmen angeordnet ist. Der Verschlusskörper weist ein seitliches Hakenelement auf, das in einem Schlitz des Druckplattenrahmens angeordnet ist. Hierdurch wird ein Kontaktpunkt gebildet, um den herum der Verschlusskörper an dem Druckplattenrahmen verschwenkt werden kann. Wenn die Kassette eingefügt und die Verschlusstür geschlossen ist, verlaufen die an der Kassette angebrachten Schläuche zwischen dem Verschlussblatt und einer Verschlussstange. Eine Schwenkbewegung des Verschlusskörpers bewegt das Verschlussblatt zu der Verschlussstange hin und von dieser weg. Sind sie zueinander mit Abstand angeordnet, wird ein ungehinderter Durchtritt der Kassettenschläuche gewährt. Liegen sie beieinander, quetschen das Verschlussblatt und die Verschlussstange die Kassettenschläuche zusammen, so dass diese verschlossen werden. Ein Vorspannen des Verschlussblattes und der Verschlussstange aufeinander zu wird durch Verschlussfedern vorgenommen. Das eine Ende der Verschlussstange ist stopfenförmig und das entsprechende Gegenstück, gegen das dieses drückt, ist rampenförmig, so dass eine schräge Quetschung des Schlauches erfolgt, was zu dessen Verletzung führen kann. Auch diese Vorrichtung erweist sich daher als nachteilig.

Eine andere Einlegevorrichtung ist in der US 5,437,635 offenbart. Auch diese Kassette weist eine Freiflussklemmeinrichtung sowie eine Flussbegrenzungseinrichtung auf. Die Flussbegrenzungseinrichtung ist vorgesehen, um den Fluss durch die entnehmbare Kassette vollständig zu stoppen. Dabei kann eine Schlauchklemme elektrisch geöffnet und geschlossen werden. Die Freiflussklemmeinrichtung ist Teil der Flussbegrenzungseinrichtung und ist an der Auslassseite des Schlauches angeordnet. Durch manuelles Bewegen der Freiflussklemmeinrichtung in die geschlossene Position kann die Kassette aus der Infusionspumpe entnommen werden. Die Freiflussklemmeinrichtung umgibt den Schlauch allseitig, wobei dieser in einem Langloch innerhalb der Einrichtung angeordnet ist. Das Langloch endet in einem schmalen Schlitz. Das Auslösen der Klemmung erfolgt dabei dadurch, dass die Flussklemmeinrichtung so weit nach außen gezogen wird, dass der Schlauch in den schmalen Schlitz hineingezogen und durch diesen geklemmt wird. Der Nachteil auch dieser Klemmvorrichtung besteht darin, dass der Schlauch durch das Hineinziehen in den schmalen Schlitz und die starke Quetschung in diesem selbst verletzt werden kann.

Aus der US 4,689,043 ist ebenfalls eine Klemmvorrichtung für einen Pumpenschlauch bekannt. Diese ist ähnlich aufgebaut wie die vorgenannte. Bei dieser wird der Schlauch ebenso durch ein Langloch geführt, das in einem schmalen Schlitz endet. Zum Quetschen wird die Flussklemme nach oben gezogen, so dass der Schlauch in den schmalen Schlitzbereich wandert und dort gequetscht wird. Es ergeben sich dabei dieselben Nachteile wie bei der vorstehend genannten Einrichtung.

Eine weitere Alternative einer Einlegevorrichtung für eine Infusionspumpe ist aus der EP 0 697 898 B1 bekannt. Die Einlegevorrichtung weist Ausnehmungen auf, in die der Infusionsschlauch eingelegt ist. Es ist außerdem eine Freiflussklemmeinrichtung vorgesehen, die L-förmig ausgebildet ist und mit einem u-förmigen Teilstück den Schlauch umgibt. An beiden Enden des u-förmigen Teilstücks sind in einem Winkel von etwa 90° längliche Schenkel angeordnet, die zum Erzeugen einer Schwenkbarkeit an ihren Enden Durchgangsöffnungen aufweisen. In diese greift ein Stift ein, um das Verschwenken zu ermöglichen. Der Querbalken des u-förmigen Teilbereichs drückt zum Klemmen den Schlauch gegen ein weiteres Teilstück, das innerhalb des u-förmigen Teilbereichs angeordnet ist. Der Klemmvorgang wird durch Verschieben eines Schiebers und durch Angreifen eines Stiftes an den Schenkeln der Freiflussklemmeinrichtung und deren Ausschwenken verursacht. Beim Zurückschieben des Schiebers wird der Schenkel wieder freigelassen und der Durchfluss durch den Schlauch aufgrund der Rückstelleigenschaften des Schlauchmaterials selbst wieder eingeleitet. Als nachteilig bei dieser Ausführungsform erweist es sich, dass zum einen die Freiflussklemmeinrichtung vergleichsweise groß ist und das Vorsehen eines Schiebers erforderlich ist. Zum anderen erfolgt wiederum eine Klemmung des Schlauchs gegen eine verhältnismäßig schmale kantige Fläche, so dass ein Verletzen von diesem nicht ausgeschlossen werden kann.

Aus der US 5,257,978 ist eine Klemmeinrichtung bekannt, bei der ein verschiebbares Element mit einem Federelement verbunden ist, das sich sowohl in Längsrichtung eines zu klemmenden Schlauchs als auch parallel und quer zu diesem erstreckt und einen abgebogenen, unter den Schlauch greifenden Abschnitt zum Klemmen aufweist.

Das Dokument US 5,437,642 offenbart eine Vorrichtung zur Vermeidung eines unerwünschten freien Flusses eines Fluids durch einen IV-Schlauch, wie er beispielsweise bei Infusionspumpen verwendet wird. Die Vorrichtung besteht aus einer Federklemme mit einer Basis und einem Federarm und einem Kanal zur Aufnahme des IV-Schlauchs. Mittels des Federarms kann das Lumen des IV-Schlauchs abgedrückt werden, um ein freien Fluss eines Fluids durch den IV-Schlauch zu verhindern. Die Vorrichtung umfasst weiterhin Mittel zum Bewegen des Federarms in der Infusionspumpe.

In dem Dokument US 4,689,043 ist eine Anordnung zum Kuppeln eines Intravenös-Schlauches mit einer Vorrichtung für die Infusion einer medizinischen Lösung in einen Patienten offenbart, wobei die Anordnung umfasst: ein mit dem Intravenös-Schlauch kuppelbares Klemmstück, das relativ zum Schlauch zwischen einer Offenstellung, in welcher Fluid durch den Schlauch strömen kann, und einer Schließstellung, in welcher der Schlauch durch das Klemmstück verschlossen ist, bewegbar ist; Mittel um den Intravenös-Schlauch im betrieblichen oder wirkungsmäßigen Eingriff mit der Vorrichtung zu halten; und eine Einrichtung, die der Vorrichtung zugeordnet werden kann und die zum Angriff am Klemmstück bewegbar ist, um das Klemmstück zwischen der Offenstellung und der Schließstellung zu verlagern; wobei das Klemmstück mit dem Intravenös-Schlauch, diesen umschließen, kuppelbar oder in Eingriff bringbar und tropfenförmig ausgebildet ist, sowie weiterhin umfassend eine an der Vorrichtung anbringbare Sperreinrichtung, um das Klemmstück bei der anfänglichen Kupplung des intravenös Schlauches mit der Vorrichtung in die Schließstellung zu stellen.

Der vorliegenden Erfindung liegt daher die Aufgabe zugrunde, eine Einlegevorrichtung für eine Pumpvorrichtung gemäß dem vorstehend genannten Stand der Technik dahingehend weiterzubilden, dass die entsprechenden genannten Nachteile des Standes der Technik vermieden werden und eine leitungs- bzw. schlauchschonende Freiflussklemmeinrichtung an der Einlegevorrichtung vorgesehen werden kann, wobei die Einlegevorrichtung einfach handhabbar und schnell in die Pumpvorrichtung einlegbar und aus dieser wieder entfernbar sein soll.

Diese Aufgabe wird durch eine Einlegevorrichtung unter anderem dadurch gelöst, dass die zumindest eine Freiflussklemmeinrichtung ein einteiliges Element aus federelastischem Material mit einem an der Einlegevorrichtung angreifenden Halteteilstück, einem auf die Leitung einwirkenden Mittelstück und einem Betätigungsteilstück aufweist, wobei das Element so angeordnet ist, dass zwischen diesem und einer Wandung der Einlegevorrichtung eine Durchführung für die Leitung verbleibt. Weiterbildungen der Erfindung sind in den abhängigen Ansprüchen definiert.

Dadurch wird eine Einlegevorrichtung geschaffen, die vergleichsweise einfach aufgebaut ist, keine zusätzlichen beweglichen Elemente aufweist, die das Einfügen und Herausholen der Einlegevorrichtung aus der Pumpvorrichtung erschweren können und die vorteilhaft klein und kompakt baut. Da die Freiflussklemmeinrichtung durch ein einteiliges Element aus federelastischem Material gebildet wird, das an geeigneter Stelle an die Einlegevorrichtung angeklemmt wird, ist hierdurch nicht nur eine einfache Handhabbarkeit der Freiflussklemmeinrichtung möglich, sondern eine kostensparende und zugleich schlauch- und leitungsschonende und kleinbauende Lösung geschaffen im Unterschied zur Vorrichtung nach EP 0 813 430. Im Unterschied zum Stand der Technik erfolgt über das Element aus federelastischem Material eine großflächige Klemmung der Leitung, so dass ein Abquetschen von dieser nicht zu befürchten steht. Für die Freiflussklemmeinrichtung reicht vorteilhaft das Element aus federelastischem Material aus, so dass kein weiteres Element, wie im Stand der Technik z.B. der EP 0 813 430 B1 erforderlich ist. Durch das Zusammenwirken mit einer Wandung der Einlegevorrichtung im Bereich der Durchführung der Leitung durch die Einlegevorrichtung wird eine direkte Klemmung möglich, da keine Kraftumlenkung, wie insbesondere bei der
EP 0 697 898 B1 offenbart, vorgenommen wird. Die Kompaktheit der Einlegevorrichtung wird durch die Freiflussklemmeinrichtung noch verstärkt, da diese die Leitung zusätzlich an der Einlegevorrichtung fixiert. Diese Fixierung und damit Sicherung besteht auch bereits ohne ein Klemmen der Leitung. Außerdem ist gemäß der EP 0 813 430 die Freiflussklemmeinrichtung vor dem Pumpsegment und Sensoren etc. angeordnet, wohingegen gemäß der vorliegenden Erfindung die Freiflussklemmeinrichtung vor dem Patienten, aber nach dem Pumpsegment angeordnet ist.

Die Freiflussklemmeinrichtung wirkt durch Zusammenwirken des einteiligen Elementes aus federelastischem Material und einer Wandung der Einlegevorrichtung, wobei zwischen die Wandung und das einteilige Element die Leitung eingelegt wird. Das einteilige Element aus federelastischem Material wird über das Halteteilstück an der Einlegevorrichtung befestigt. Diese Befestigung wird vorteilhaft auch über das Zusammenwirken des Halteteilstücks und des Betätigungsteilstücks durch ein Festklemmen an der Einlegevorrichtung erzeugt. Das Betätigungsteil stück ist zum Lösen der Klemmung einer Leitung vorgesehen. Das Mittelstück des einteiligen Elementes aus federelastischem Material wirkt direkt auf die Leitung ein. Es ist daher so angeordnet, dass die Leitung zwischen dem Mittelstück und der Wandung der Einlegevorrichtung eingefügt werden kann.

Vorzugsweise ist das Betätigungsteilstück so in der Einlegevorrichtung und/oder in einem weiteren Element angeordnet oder anordbar, dass es und/oder dieses Element von außerhalb der Einlegevorrichtung zugänglich ist, insbesondere für das Einwirken zumindest eines Teils einer Verschlussklappe der Pumpvorrichtung zum Lösen und Auslösen einer Klemmung. Es reicht somit vorteilhaft allein die Anordnung des Elements aus federelastischem Material in der Einlegevorrichtung aus, um ein Lösen und Auslösen einer Klemmung der Leitung, also ein Schaffen einer Freiflussposition und einer Freiflussklemmung zu ermöglichen. Es sind keine weiteren Elemente oder Einrichtungen erforderlich, um eine Freiflussklemmung auszulösen, wenn beispielsweise die Verschlussklappe der Pumpvorrichtung geöffnet wird. Die Verschlussklappe kann so gegen das Betätigungsteilstück drücken, dass das federnde Element von der Wandung der Einlegevorrichtung weggedrückt und die Durchführung für die Leitung offengehalten wird. Aufgrund dieser Anordnung der Betätigungseinrichtung erfolgt ein automatischer Verschluss der Leitung bei Öffnen der Verschlussklappe, was einen erheblichen Sicherheitsaspekt darstellt. Es könnte ansonsten bei Öffnen der Verschlussklappe eine undefinierte Menge an Medium durch die Leitung fließen, was ein großes Risiko für den Patienten darstellen kann. Sobald die Verschlussklappe der Pumpvorrichtung geöffnet wird, wird jedoch sofort der Austritt von Medium aus und/oder die Förderung durch die Leitung aufgrund des Klemmens der Leitung eingestellt.

Soll beim ersten Einlegen der Einlegevorrichtung und ggf. bei geöffneter Verschlussklappe der Pumpvorrichtung eine Freiflussposition erreicht werden, kann die Klemmwirkung einer Freiflussklemmeinrichtung durch zumindest eine Freiflussarretiereinrichtung aufgehoben und eine Freiflussposition arretiert werden. Auch ein Drücken gegen ein Betätigungsteilstück der Freiflussklemmeinrichtung per Hand ist möglich, wenn die Verschlussklappe der Pumpvorrichtung geöffnet ist, um die Klemmwirkung aufzuheben und einen Freifluss zu erreichen. Besonders bevorzugt weist die zumindest eine Freiflussarretiereinrichtung ein Griffelement und ein zwischen das Element aus federelastischem Material und die Einlegevorrichtung einfügbares Arretierelement auf. Bevorzugt ist das Arretierelement stiftförmig. Es kann ggf. außerdem mit einem auskragenden Ende versehen sein. Die Freiflussarretiereinrichtung kann somit bei aus der Pumpvorrichtung herausgenommener Einlegevorrichtung eine Freiflussposition schaffen, die ansonsten aufgrund der Vorspannung des federelastischen Elementes eine Klemmung einer eingelegten Leitung vornimmt. Diese Klemmposition kann durch Ziehen an dem Griffelement, das mit dem Arretierelement verbunden ist, schnell hervorgerufen werden, da dadurch das Arretierelement zwischen der Betätigungseinrichtung und einer Wandung der Einlegevorrichtung herausgezogen wird. Durch Vorsehen eines stiftförmigen Arretierelementes mit einem auskragenden Ende wird ein verbesserter Halt und zugleich eine Möglichkeit geschaffen, um ein ungewolltes Herausziehen des Arretierelementes zu verhindern, da das Herausziehen des auskragenden Endes eines größeren Kraftaufwands bedarf als beispielsweise das Herausziehen eines durchgängig stiftförmigen Elementes. Nach dem Entfernen der Freiflussarretiereinrichtung kann eine Freiflussposition zwar grundsätzlich durch Wiedereinfügen einer Freiflussarretiereinrichtung hergestellt werden. Üblicherweise erweist sich dies jedoch als schwierig, so dass zumeist von Hand eine Freiflussposition erzeugt wird, wenn dies nach dem Herausnehmen der Einlegevorrichtung gewünscht wird.

Bevorzugt greift das Halteteilstück der Freiflussklemmeinrichtung in eine Aussparung oder Ausnehmung, insbesondere einen Schlitz, der Einlegevorrichtung so weit ein, dass zumindest das Betätigungsteilstück sich klemmend an der Einlegevorrichtung anlegt oder in ein Arretierstück eingreift. Durch den Eingriff in eine Aussparung oder Ausnehmung bzw. Öffnung der Einlegevorrichtung wird ein besserer Halt an dieser ermöglicht als dies beispielsweise durch bloßes Umgreifen einer Wandung der Einlegevorrichtung möglich ist. Allerdings ist es dennoch möglich, je nach Ausbildung der Einlegevorrichtung auch lediglich ein solches Umgreifen eines Teils der Einlegevorrichtung mit dem Halteteilstück und Befestigen des Elementes aus federelastischem Material an der Einlegevorrichtung durch Wirken einer Klemmkraft zwischen dem Halteteilstück und dem Betätigungsteilstück zu ermöglichen. Ein einfaches Anklipsen des einteiligen Elementes aus federelastischem Material ist in jedem der Fälle vorteilhaft möglich, was unter anderem die Montage erleichtert.

Die Einlegevorrichtung weist im Bereich der Leitungsdurchführung zumindest eine Öffnung zum Einfügen einer Sensoreinrichtung und
zumindest eine weitere Öffnung zum Eingreifen einer Antriebseinrichtung der Pumpvorrichtung auf, wobei die Freiflussklemmeinrichtung zwischen der Öffnung für die Antriebseinrichtung und einer Öffnung für einen Drucksensor angeordnet ist. Hierdurch kann über den Drucksensor direkt erfasst werden, ob die Freiflussklemmeinrichtung angesprochen hat, also beispielsweise versehentlich die Verschlussklappe der Pumpvorrichtung geöffnet wurde. Durch die Druckveränderung innerhalb der Lei-tung kann dann ein entsprechender Alarm ausgelöst, die Antriebseinrichtung abgestellt oder anderweitig reagiert werden.

Vorzugsweise ist eine Verpolungsschutzeinrichtung zum Kennzeichnen der Einlegerichtung der Einlegevorrichtung in der Pumpvorrichtung vorgesehen. Besonders bevorzugt ist die Verpolungsschutzeinrichtung eine ungleichmäßige Formgebung der Einlegevorrichtung, insbesondere ist ein Stegelement oder eine andere Einrichtung lediglich einseitig vorgesehen. Um die Anordnung der Freiflussklemmeinrichtung patientenseitig, also hinter der Antriebseinrichtung vorzusehen, erweist es sich als vorteilhaft, wenn die Einlegevorrichtung so ausgebildet ist, dass sie nur in einer Richtung in die Pumpvorrichtung eingelegt werden kann. Das Vorsehen einer ungleichmäßigen Formgebung erweist sich dabei als die kostengünstigste Möglichkeit einer Verpolungsschutzeinrichtung. Alternativ oder zusätzlich können ein oder mehrere weitere Elemente vorgesehen werden, die lediglich einseitig und/oder bei einer bestimmten Ausführungsform vorgesehen sind. Solche Elemente können z.B. in Öffnungen eingefügte Stößel oder Stopfen sein, die die Öffnungen verschließen und dadurch eine diesbezügliche Abtastung und Unterscheidung erlauben.

Vorzugsweise ist eine ein- oder mehrteilige Leitung vorgesehen, wobei bei einer mehrteiligen Leitung Verbindungsstücke zum Verbinden der Leitungsstücke vorgesehen sind. Im Gegensatz zu den meisten Einlegevorrichtungen des Standes der Technik ist bei der vorliegenden aufgrund der an die Einlegevorrichtung anklipsbaren Freiflussklemmeinrichtung auch die
Verwendung einer einteiligen Leitung möglich, wenn diese an beiden Enden bereits mit Einrichtungen versehen ist, wie beispielsweise einem Infusionsbeutel und einem Infusionsbesteck. Bei nahezu allen Einlegevorrichtungen des vorstehend zitierten Standes der Technik ist das Durchführen der Leitung durch die Freiflussklemmeinrichtung erforderlich. Bei der vorliegenden Erfindung ist dies nicht nötig, da die Freiflussklemmeinrichtung nach dem Einlegen der Leitung in die Einlegevorrichtung über diese hinweg an die Einlegevorrichtung angeklemmt werden kann. Bei Bedarf kann jedoch auch eine mehrteilige Leitung verwendet werden.

Besonders bevorzugt bestehen ein oder mehrere aus der Einlegevorrichtung herausgeführte Leitungsstücke aus einem harten, starren, knickfesten Material. Das innerhalb der Einlegevorrichtung liegende Leitungsstück besteht demgegenüber vorzugsweise aus einem weicheren Material, damit die Antriebseinrichtung der Pumpvorrichtung ein kontinuierliches Verformen der Leitung zum Fördern des darin fließenden Mediums ermöglichen kann. Die vorzugsweise vorgesehenen Leitungsverbindungsstücke können in bevorzugt vorgesehene, entsprechend geformte Ausnehmungen innerhalb der Einlegevorrichtung verrutschsicher eingelegt werden. Hierdurch wird die Leitung ortsfest fixiert und eine stabile Einheit geschaffen.

Dies erweist sich auch als besonders vorteilhaft bei der bevorzugten Verwendung einer Mehrkanalumschalteinrichtung. Eine solche Mehrkanalumschalteinrichtung kann als alternative Ausführungsform zu einer Einkanalvariante oder als mit einer solchen verbindbares Element vorgesehen werden. Grundsätzlich kann eine einkanalige und/oder eine mehrkanalige Einlegevorrichtung vorgesehen sein. Es können also separate einkanalige und mehrkanalige Varianten ausgebildet werden. Zusätzlich ist es möglich, eine mit einer einkanaligen Einlegevorrichtung verbindbare oder verbundene Mehrkanalumschalteinrichtung vorzusehen. Bei der Verwendung einer mit der Einlegevorrichtung verbindbaren Mehrkanalumschalteinrichtung ist es wichtig, dass das Auslassende der Mehrkanalumschalteinrichtung exakt auf die durch die Einlegevorrichtung gehende Leitung aufgefügt wird. Zu diesem Zweck erweist es sich als vorteilhaft, wenn die Mehrkanalumschalteinrichtung vorzugsweise zumindest ein Verbindungselement, insbesondere einen Verbindungsabschnitt nach Art eines Ventilkörpers und die Einlegevorrichtung Nuten, Öffnungen, Aussparungen oder dergleichen Einrichtungen zum Einfügen des Verbindungselements aufweist. Ebenso ist eine umgekehrte Anordnung von Verbindungselementen an der Einlegevorrichtung und Nuten, Öffnungen etc. an der Mehrkanalumschalteinrichtung möglich. Hierdurch wird in jedem der Fälle eine exakte Ausrichtung zu der durch die Einlegevorrichtung gehenden Leitung sowie zu der Einlegevorrichtung selbst möglich. Eine auswechselbare Mehrkanalumschalteinrichtung und/oder Einkanalvariante erweist sich im Hinblick auf die vielseitige Verwendbarkeit der Pumpvorrichtung als besonders vorteilhaft. Durch Einlegen einer unterschiedlichen Anzahl von Zuleitungen in den die Mehrkanalumschalteinrichtung darstellenden Teil können unterschiedliche Ausführungsformen erzielt werden. Z.B. ist auch lediglich das Vorsehen einer Zuleitung möglich, wodurch eine einkanalige Variante mit der für den Mehrkanalbereich geeigneten Ausführung möglich ist.

Vorzugsweise sind Sensor erfassbare Teilbereiche der Einlegevorrichtung zum Unterscheiden einer ein- von einer mehrkanaligen Einlegevorrichtung unterschiedlich ausgeführt, insbesondere mit unterschiedlichen Dicken und/oder unterschiedlich angeordneten Absätzen versehen. Die ein- und die mehrkanalige Einlegevorrichtung können im übrigen im Wesentlichen dieselbe Form aufweisen und nur im Bereich des die Mehrkanalumschalteinrichtung aufweisenden Endes anders ausgeführt sein. Um eine möglichst kostengünstige Fertigung der verschiedenen Varianten von Einlegevorrichtungen zu ermöglichen, bestehen vorzugsweise die Unterschiede nur in Bezug auf die Dickenabmessungen bzw. zusätzliche Absätze, so dass im Wesentlichen dasselbe Werkzeug für die Herstellung der Einlegevorrichtungen verwendet werden kann. Unterschiedliche Dickenabmessungen bzw. zusätzliche Absätze oder Elemente, wie z.B. Stößel etc., können leicht durch entsprechende Sensoren der Pumpvorrichtung erfasst werden, so dass ein Steuerungsprogramm der Pumpvorrichtung nach dem Erkennen der Einlegevorrichtungsart die jeweils erforderliche Ansteuerung, insbesondere auch der Mehrkanalumschalteinrichtung vornimmt.

Zum Arretieren einer Freiflussposition kann bevorzugt im Bereich der Mehrkanalumschaltvorrichtung eine weitere Freiflussarretiereinrichtung vorgesehen sein, die mit zumindest einer Freiflussklemmeinrichtung zusammen wirkt. Hierdurch können einzelne oder alle Zuleitungen im Freifluss arretiert werden. Die Ausbildung der Freiflussarretiereinrichtung entspricht vorzugsweise der der vorstehend für das andere Ende der Einlegevorrichtung beschriebenen Freiflussarretiereinrichtung.

Bevorzugt ist ein Filterelement mit der Leitung verbindbar oder so angeordnet, dass in der Leitung befindliches Medium zum Entlüften durch das Filterelement hindurch und von diesem zurück an einer Sensoreinrichtung vorbeileitbar ist. Besonders bevorzugt ist das Filterelement außerhalb der Einlegevorrichtung und die Sensoreinrichtung im Bereich der Einlegevorrichtung angeordnet. Das in der Leitung befindliche bzw. durch diese hindurch beförderte Medium wird somit aus der Einlegevorrichtung heraus zu dem außerhalb der Einlegevorrichtung befindlichen Filterelement befördert, in diesem entlüftet und wieder zu der Einlegevorrichtung zurückbefördert. Dort wird es an einer Sensoreinrichtung vorbei befördert, die den Luftgehalt des Mediums detektiert und gegebenenfalls den Pumpvorgang durch Abgabe eines entsprechenden Signals stoppen kann, für den Fall, dass sich zu viel Luft in dem Medium befindet, was eine Gefährdung des Lebens des Patienten zur Folge haben könnte. Die Sensoreinrichtung im Bereich der Einlegevorrichtung vorzusehen, weist den besonderen Vorteil auf, dass diese geschützt und an definierter Stelle an der Einlegevorrichtung angeordnet werden kann, so dass ein Zugriff auf die Daten der Sensoreinrichtung direkt von einer Steuerungseinrichtung zum Steuern des Pumpvorgangs innerhalb der Pumpvorrichtung aufgenommen werden kann. Auch das Vorsehen des Filterelements außerhalb der Einlegevorrichtung bzw. Pumpvorrichtung erweist sich als vorteilhaft, da hierdurch das Filterelement leichter ausgetauscht werden kann, wenn dies erforderlich werden sollte. Außerdem ist auch das Entlüften einfacher möglich, wenn sich das Filterelement außerhalb der Einlegevorrichtung befindet.

Vorzugsweise ist das Element aus federelastischem Material im Wesentlichen C-förmig mit einem abgespreizten Endstück. Aufgrund der C-Form ist zum einen eine formbegründete Vorspannung zwischen dessen beiden Endbereichen gegeben. Zum anderen ist das Umgreifen der Einlegevorrichtung problemlos möglich. Außerdem wird durch die C-Form die Federelastizität noch verstärkt und das Element als solches stabiler. Das bevorzugte abgespreizte Endstück erweist sich zum Eingreifen in ein Arretierstück und/oder Angreifen durch beispielsweise eine Verschlussklappe einer Pumpvorrichtung als besonders vorteilhaft, da dieses aus der Einlegevorrichtung nach außen ragen kann. Auch die Herstellung eines im Wesentlichen C-förmigen Elementes mit abgespreiztem Endstück ist gegenüber anderen Formgebungen, die ebenfalls verwendet werden können, leichter und kostengünstiger herzustellen.

Bevorzugt wird als federelastisches Material Federstahl oder ein anderes Rückstelleigenschaften aufweisendes Material verwendet. Im Gegensatz zu den aufwändigen und teilweise aus Kunststoff gefertigten Freiflussklemmeinrichtungen des Standes der Technik ist die bevorzugte Verwendung von Federstahl nicht nur kostengünstiger, sondern weist auch im Allgemeinen eine längere Lebensdauer auf. Gerade die Verwendung eines Kunststoffs in Verbindung mit Spiralfedern, wie dies in EP 0 813 430 vorgeschlagen wird, birgt die Gefahr eines Brechens aufgrund Versprödung der Kunststoffteile in sich. In einem solchen Falle wird dann entweder keine Klemmung oder eine sehr starke Quetschung und möglicherweise Durchtrennung der Leitung verursacht. Dies geschieht vorteilhaft mit der Freiflussklemmeinrichtung gemäß der vorliegenden Erfindung nicht mehr.

Das in der US 5,257,978 offenbarte Sicherheitsmodul wird außerhalb einer Pumpe angeordnet. Die Größenabmessungen des Sicherheitsmoduls sind gegenüber denen der erfindungsgemäßen Einlegevorrichtung viel größer, so dass das Sicherheitsmodul auch nicht in eine Pumpvorrichtung eingelegt werden kann. Die erfindungsgemäße Freiflussklemmeinrichtung ist im Unterschied zu dem Sicherheitsmodul gemäß der US 5,257,978 nach einer Pumpvorrichtung patientenseitig angeordnet. Sie ist ein abnehmbarer, aber zu der Einlegevorrichtung gehöriger und somit fester Bestandteile von dieser. Die erfindungsgemäße Einlegevorrichtung ist Teil der Pumpvorrichtung, da Lamellen der Pumpvorrichtung in entsprechende Öffnungen bzw. eine entsprechende Öffnung der Einlegevorrichtung, in der eine Leitung bzw. ein Schlauch liegt, eingreifen, um an diesem anzugreifen und die Pumpbewegung für das in dem Schlauch befindliche Medium hervorrufen zu können. Die erfindungsgemäße Einlegevorrichtung wird vollständig mit der erfindungsgemäßen Freiflussklemmeinrichtung versehen vorbereitet und kann als Gesamtheit in eine Pumpvorrichtung eingelegt und wieder aus dieser herausgenommen werden. Als besonders vorteilhaft erweist es sich dabei, dass automatisch die Freiflussklemmeinrichtung den Schlauch klemmt, wenn eine Verschlussklappe der Pumpvorrichtung geöffnet wird und nach deren Schließen bzw. Einlegen der Einlegevorrichtung in die Pumpvorrichtung und gegebenenfalls Entfernen einer Freiflussarretiereinrichtung die gesamte Pumpvorrichtung einsatzbereit ist. Eine Manipulation an einem Schieber, wie dies in der US 5,257,978 vorgesehen ist, ist bei der erfindungsgemäßen Einlegevorrichtung nicht erforderlich. Aufgrund der Verwendung von besonders starren Materialien für die Leitung bzw. den Schlauch, der durch die Einlegevorrichtung hindurchgeht, um ein Knicken und Okklusionen zu vermeiden, erweist es sich bei der erfindungsgemäßen Einlegevorrichtung als besonders vorteilhaft, dass die Freiflussklemmeinrichtung nicht außerhalb der Pumpvorrichtung vorgesehen ist, was aufgrund der starren Materialien des Schlauchs bzw. der Leitung auch nicht möglich wäre. Bei dem Sicherheitsmodul gemäß der US 5,257,978 muss der Patient bzw. das Krankenhauspersonal stets gezielt eine Klemmung des Schlauchs hervorrufen, wohingegen bei der erfindungsgemäßen Einlegevorrichtung eine solche Klemmung automatisch erfolgt und aufgehoben wird, beliebig häufig und stets dann, wenn dies erforderlich ist bzw. automatisch bei Öffnen einer Pumpvorrichtung und deren Schließen bzw. Einlegen und Herausnehmen der Einlegevorrichtung. Die erfindungsgemäße Einlegevorrichtung und insbesondere die erfindungsgemäße Freiflussklemmeinrichtung weisen daher eine eindeutige Funktion auf, die ohne zusätzliche Maßnahmen oder Manipulationen selbstständig abläuft, wodurch die Verwendung der erfindungsgemäßen Einlegevorrichtung und von entsprechend ausgestatteten Pumpvorrichtungen für einen Patienten einfach und unkompliziert abläuft, ohne dass dieser selbst tätig werden muss. Aufgrund der durch die erfindungsgemäße Einlegevorrichtung mit erfindungsgemäßer Freiflussklemmeinrichtung mögliche kleine Bauform erweist sich auch die Handhabung der Einlegevorrichtung als besonders einfach und daher für die ambulante, mobile Anwendung besonders geeignet, wohingegen das in der US 5,257,978 beschriebene Sicherheitsmodul gerade für große stationäre Pumpeinrichtungen ausgebildet ist.

Zur näheren Erläuterung der Erfindung werden im folgenden Ausführungsbeispiele anhand der Zeichnungen näher beschrieben. Diese zeigen in:
- Fig. 1: eine perspektivischer Draufsicht auf ein Gehäuse einer Pumpvorrichtung mit einer peristaltischen Antriebseinrichtung und einer eingelegten erfindungsgemäßen Einlegevorrichtung,
- Fig. 2: eine Draufsicht auf das Gehäuse der Pumpvorrichtung gemäß Fig. 1,
- Fig. 3: eine perspektivische Ansicht einer ersten Ausführungsform einer einkanaligen erfindungsgemäßen Einlegevorrichtung,
- Fig. 4: eine perspektivische Ansicht der Rückseite der Einlegevorrichtung gemäß Fig. 3,
- Fig. 5: eine Explosionsansicht der Einlegevorrichtung gemäß Fig. 3,
- Fig. 6: eine Querschnittsansicht durch die Einlegevorrichtung gemäß Fig. 3 bis 5 im Bereich der Freiflussklemmeinrichtung,
- Fig. 6a: eine Querschnittsansicht durch eine andere erfindungsgemäße Einlegevorrichtung im Bereich der Freiflussklemmeinrichtung,
- Fig. 7: eine Draufsicht auf die Einlegevorrichtung gemäß Fig. 3,
- Fig. 8: eine Seitenansicht der Einlegevorrichtung gemäß Fig. 3,
- Fig. 9: eine perspektivische Ansicht einer zweiten Ausführungsform einer erfindungsgemäßen mehrkanaligen Einlegevorrichtung,
- Fig. 10: eine perspektivische Ansicht der Rückseite der mehrkanaligen Einlegevorrichtung gemäß Fig. 9,
- Fig. 11: eine Explosionsansicht der mehrkanaligen Einlegevorrichtung gemäß Fig. 9,
- Fig. 12a und 12b: zwei Seitenansichten der mehrkanaligen Einlegevorrichtung gemäß Fig. 9,
- Fig. 13: eine Draufsicht auf die mehrkanalige Einlegevorrichtung gemäß Fig. 9,
- Fig. 14a und 14b: Schnittansichten durch einkanalige und mehrkanalige Einlegevorrichtungen im Vergleich,
- Fig. 15: eine perspektivische Ansicht einer dritten Ausführungsform einer einkanaligen erfindungsgemäßen Einlegevorrichtung mit Filterelement,
- Fig. 16: eine perspektivische Ansicht der Rückseite der einkanaligen Einlegevorrichtung gemäß Fig. 15,
- Fig. 17: eine perspektivische Explosionsansicht der einkanaligen Einlegevorrichtung mit Filterelement gemäß Fig. 15 und 16,
- Fig. 18: eine bemaßte Rückansicht der Einlegevorrichtung gemäß Fig. 15 und 16,
- Fig. 19: eine perspektivische Explosionsansicht einer weiteren Ausführungsform einer erfindungsgemäßen mehrkanaligen Einlegevorrichtung mit Filterelement,
- Fig. 20: eine perspektivische Zusammenbauansicht der mehrkanaligen Einlegevorrichtung gemäß Fig. 19,
- Fig. 21: eine perspektivische Rückansicht der Einlegevorrichtung gemäß Fig. 20,
- Fig. 22: eine bemaßte Rückansicht der Einlegevorrichtung gemäß Fig. 20 und 21, und
- Fig. 23: eine Querschnittsansicht durch eine weitere Ausführungsform einer Einlegevorrichtung gemäß der vorliegenden Erfindung im Bereich der Freiflussklemmeinrichtung.

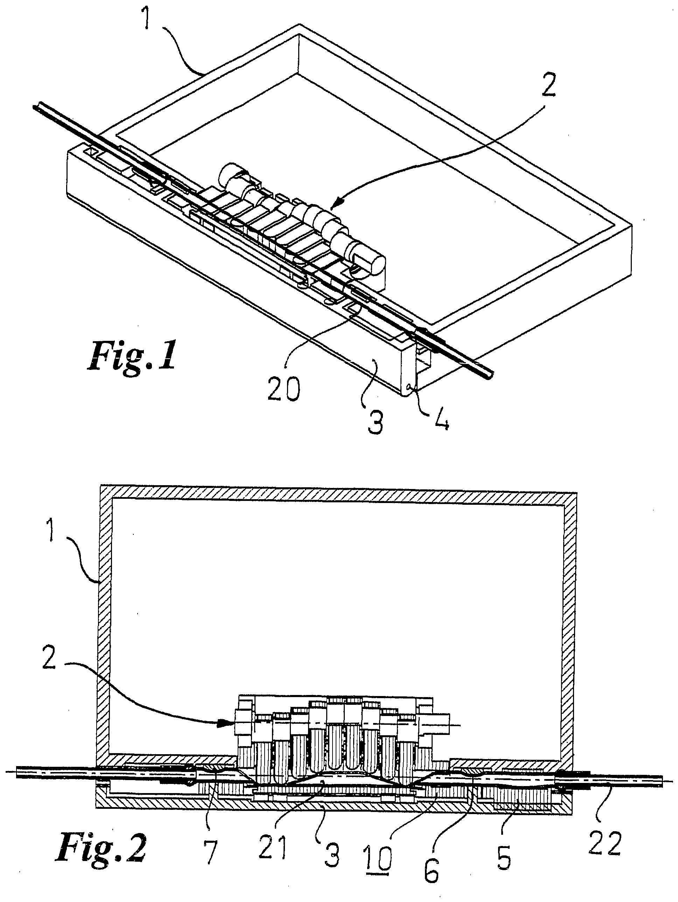

Fig. 1 zeigt eine perspektivische Ansicht eines Gehäuses 1 einer Pumpvorrichtung, von der lediglich eine peristaltische Antriebseinrichtung 2 dargestellt ist. Die übrigen Teile, wie insbesondere Motor und Steuerungseinrichtung sind der Übersichtlichkeit halber weggelassen. Das Gehäuse 1 weist auf seiner einen Seite eine Verschlussklappe 3 auf. Diese ist in Fig. 1 geschlossen dargestellt. Über ein Scharniergelenk 4 kann sie aufgeklappt und eine darin befindliche Einlegevorrichtung 10 (siehe Fig. 2) entnommen werden. In die Einlegevorrichtung ist eine Leitung 20 eingelegt. Diese kann beispielsweise ein flexibler Schlauch sein.

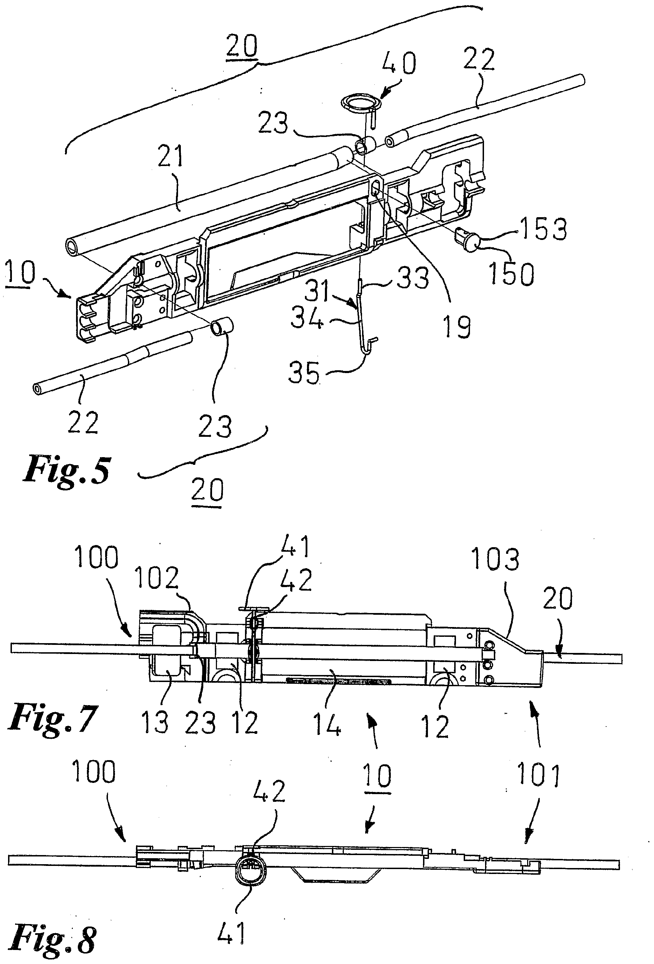

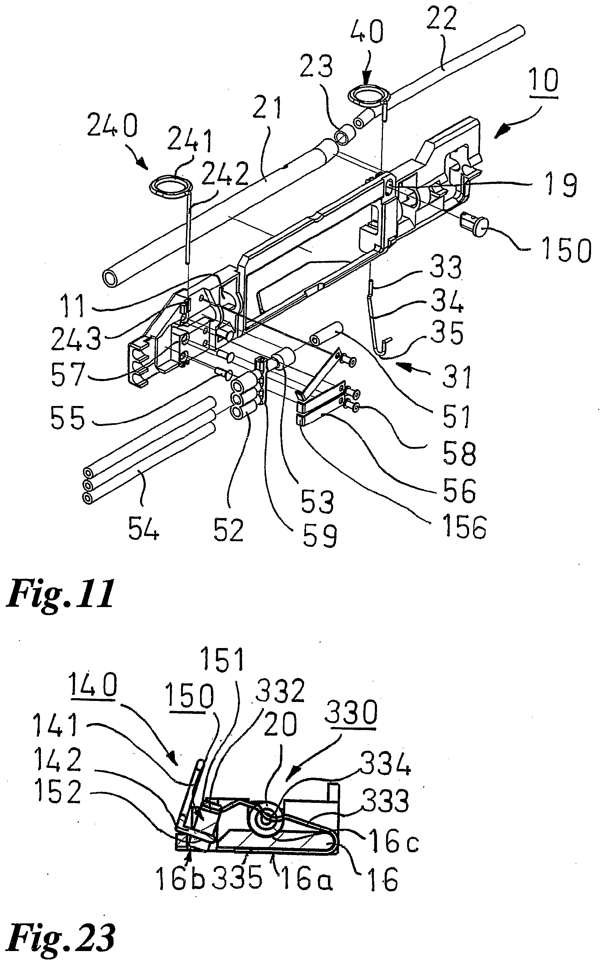

Wie besser Fig. 2 entnommen werden kann, greifen in die Einlegevorrichtung 10 Luft- und Drucksensoren 5, 6, 7 ein. Die beiden Drucksensoren 6 und 7 sind jeweils vor und hinter der peristaltischen Antriebseinrichtung angeordnet, um die Druckverhältnisse dort jeweils aktuell abfragen zu können. Patientenseitig ist außerdem der Luftsensor 5 vorgesehen. Die Leitung gemäß Fig. 2 ist aus drei Teilen aufgebaut, wobei ein mittleres Leitungsstück 21 bevorzugt aus einem weicheren Material besteht als die beiden Leitungsendstücke 22. Die drei Leitungsstücke sind über ein jeweiliges Verbindungsstück 23 miteinander verbunden. Gemäß Fig. 2 sind diese Verbindungsstücke in die beiden Enden des mittleren Leitungsstücks eingearbeitet. Wie insbesondere den Fig. 5 und 11 entnommen werden kann, können die Verbindungsstücke jedoch auch separat in die Leitungsenden des mittleren Leitungsstücks eingefügt und die jeweiligen Leitungsendstücke 22 dort aufgefügt werden. Um einen Schutz gegen mechanische Beschädigung und eine Knickfestigkeit zu gewähren, bestehen die Leitungsendstücke bevorzugt aus einem härteren knickfesten und starren Material. Anstelle mehrerer einzelner Leitungsstücke kann auch ein durchgehender Schlauch bzw. eine durchgehende Leitung verwendet werden. Das Erfordernis eines Vorsehens von Verbindungsstücken entfällt dann. Das mittlere Leitungsstück besteht bevorzugt aus einem flexiblen Material, um, durch die Antriebseinrichtung beaufschlagt, eine peristaltische Pumpbewegung ausüben zu können. Es können beliebige diese Flexibilität aufweisende Materialien verwendet werden. Die Leitung bzw. die Leitungsstücke können jeweils beliebige Durchmesser und Wandstärken bzw. Abmessungen aufweisen.

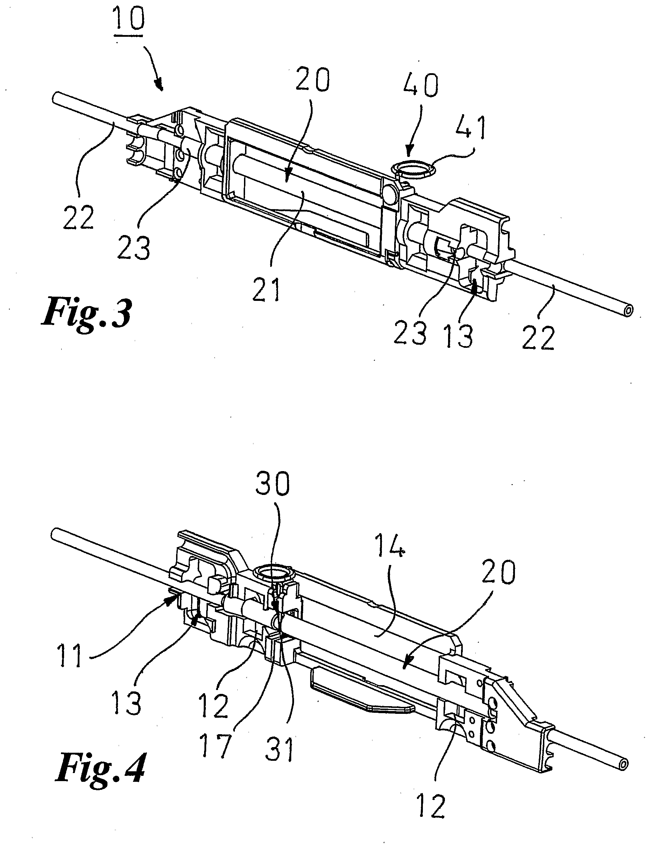

In den Fig. 3 und 4 ist eine perspektivische Ansicht einer ersten Ausführungsform einer einkanaligen Einlegevorrichtung gezeigt. Wie insbesondere Fig. 4 entnommen werden kann, weist die Einlegevorrichtung in Längsrichtung Ausnehmungen, Aussparungen bzw. Spalte 11 auf, in die die Leitung 20 eingelegt ist. Die Leitung läuft sowohl durch Ausnehmungen 12 für die Drucksensoren als auch durch eine Aussparung 13 für den Luftsensor und eine Aussparung 14 für die Antriebseinrichtung hindurch. Die Ausnehmungen bzw. Spalte 11 können unterschiedlich breit ausgebildet sein, um die Leitung in allen Bereichen optimal aufnehmen und hinsichtlich ihrer Position festlegen zu können.

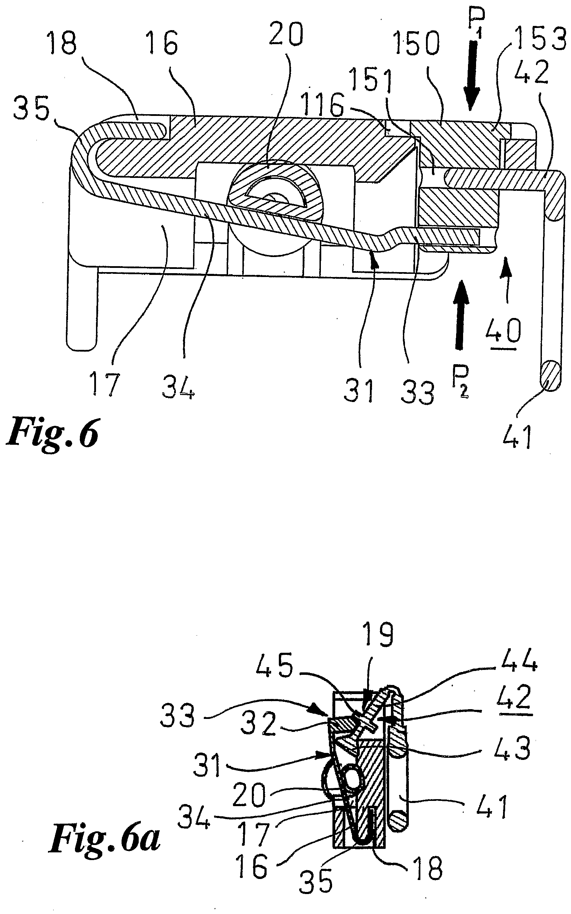

In Fig. 4 ist eine Freiflussklemmeinrichtung 30 zu sehen. Die Freiflussklemmeinrichtung weist ein Element 31 aus federelastischem Material auf, wie besser Fig. 5 zu entnehmen. Dieses ist in Fig. 4 lediglich von der Rückseite, die Leitung 20 umgebend, gezeigt. Wie besonders gut den Fig. 5 und 6 zu entnehmen, ist das Element aus federelastischem Material im wesentlichen C-förmig mit einem Betätigungsteilstück 33, das an der Einlegevorrichtung von außen zugänglich angeordnet ist. Das Betätigungsteilstück kann - wie in der dargestellten Ausführungsform - mit dem Element 31 einteilig oder als Endstück auf das Element 31 aufgefügt sein. Beispielsweise kann das Betätigungsteilstück auch als gebogenes Ende ausgebildet sein, an dem durch Druck angegriffen werden kann. An das Betätigungsteilstück 33 schließt sich ein Mittelstück 34 und an dieses ein Halteteilstück 35 an. Das gekrümmte Halteteilstück 35 umgreift auf einer dem Betätigungsteilstück gegenüberliegenden Seite der Einlegevorrichtung diese und hält klemmend dadurch das Element 31 daran fest. Das gerade Mittelstück 34 bildet dabei zwischen sich und einer Wandung 16 der Einlegevorrichtung eine Durchführöffnung bzw. ein Schlitz 17, durch die bzw. den die Leitung 20 hindurchtritt. Da diese Durchführöffnung zwischen dem Mittelstück des anklemm- bzw. anklipsbaren Elementes 31 aus federelastischem Material und der Wandung 16 gebildet wird, kann zunächst die Leitung in die Einlegevorrichtung eingelegt und nachfolgend das Element 31 auf die Einlegevorrichtung aufgeklipst werden.

Wie insbesondere Fig. 6 zu entnehmen, kann zum Erzeugen eines besseren Haltes das Halteteil stück 35 in einen Schlitz eine Aussparung etc. 18 der Einlegevorrichtung eingreifen und sich darin festhalten. Grundsätzlich kann das Halteteilstück die Wandung 16 von unten umgreifen, so dass das Fertigen eines zusätzlichen Schlitzes, einer Aussparung, Ausnehmung etc. in diesem Bereich nicht mehr erforderlich ist. Wie in Fig. 6 zu sehen, greift das Betätigungsteilstück 33 in eine Öffnung 151 eines Arretierstücks 150 ein.

Eine automatische Klemmung der Freiflussklemmeinrichtung 30 kann durch eine Freiflussarretiereinrichtung 40 verhindert werden. Die Freiflussarretiereinrichtung 40 umfasst ein Griffelement 41 in Form eines 0-Rings sowie ein Arretierelement 42, das in das Arretierstück 150 in eine darin vorgesehene Öffnung 151 eingreifen kann. Das Arretierelement ist zu diesem Zweck im Wesentlichen stiftförmig. Bei einer anderen Ausführungsform, wie sie in Figur 6a gezeigt ist, kann es ein auskragendes Ende 43 aufweisen, das mit einem abgebogenen Endstück des Betätigungsteilstücks zusammenwirkt. Es kann eine umlaufende Auskragung 45 aufweisen, an der das Endstück des Betätigungsteilstücks besser angreifen und sich zusätzlich abstützen kann. Griffelement und Arretierelement können gelenkig miteinander verbunden sein, insbesondere durch zwei ineinandergreifende Ösen. Gemäß Figur 6 sind sie jedoch einteilig als z.B. Kunststoffspritzgussteil ausgebildet. Durch Ziehen an dem Griffelement 41 kann die Freiflussposition gemäß Fig. 6 in eine Freiflussklemmposition umgeändert werden. In der klemmenden Position greift das Element 31 aus federelastischem Material, z.B. einem federelastischen Metall oder Kunststoff oder Verbundwerkstoff, hinweg an der Leitung 20 an. Da hierbei die Leitung nicht über scharfe Kanten gezwängt wird, wie es im Stand der Technik der Fall ist, kann eine Verletzung der Leitung im wesentlichen vermieden werden. Das Mittelstück 34 ist in der dargestellten Ausführungsform schmal drahtartig. Es kann alternativ aber auch breiter ausgeführt sein. Eine Beschädigung von beispielsweise sich innerhalb der Leitung befindenden Blutkörperchen durch zu starke Quetschung der Leitung kann dabei vorteilhaft verhindert werden. Insbesondere ist es auch möglich, die Wandung 16 im Bereich der Leitungsdurchführung mit einer Mulde zu versehen, in die sich die Leitung hineinlegen kann, so dass ein noch besseres Widerlager für die Leitung während des Klemmvorganges gebildet wird.

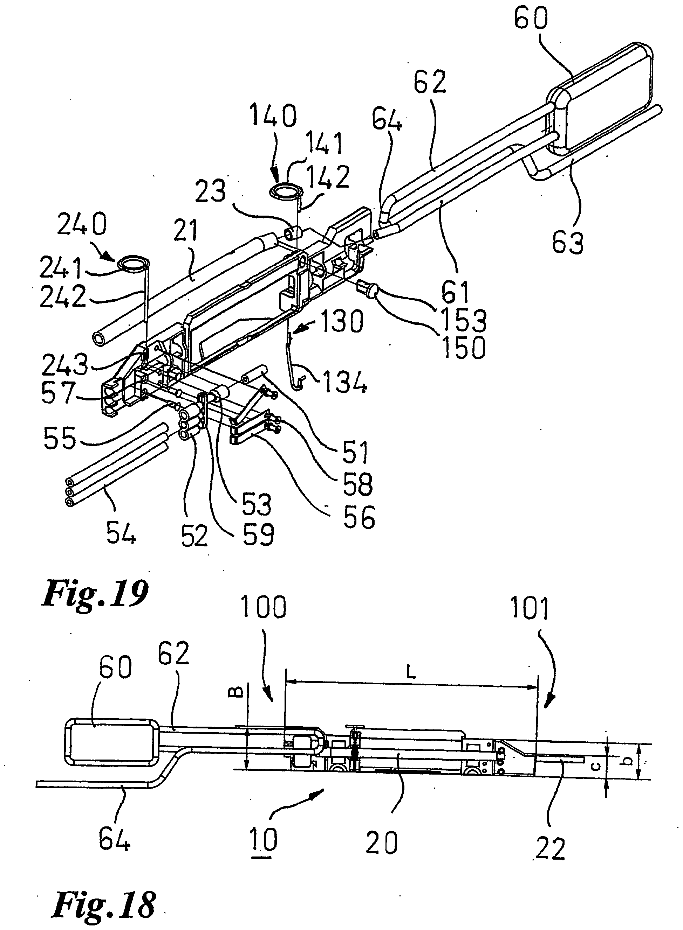

Wie insbesondere der Fig. 5 und der Fig. 19 entnommen werden kann, weist die Einlegevorrichtung zum Einfügen des Arretierstücks 150 außerdem eine durchgehende Öffnung 19 auf. Das Arretierstück 150 weist einen Endflansch 153 auf, der mit einer Außenfläche der Wandung 16 zusammenwirkt, wobei sich der Endflansch auf der Außenfläche abstützt. Zum Erzeugen einer glatten Außenfläche der Wandung, ohne Erhebungen, liegt der Endflansch in einer Ausnehmung 116. Eine solche Ausnehmung muss jedoch nicht vorgesehen sein.

Bei Verschließen der Pumpvorrichtung durch die Verschlussklappe gemäß Fig. 1 und 2 drückt diese mit einem Teilbereich in Richtung des Pfeils P₁ gegen das Arretierstück 150 und öffnet somit die Leitung 20. Durch Öffnen der Verschlussklappe springt aufgrund der Vorspannung des Elements 31 das Arretierstück 150 in Richtung des Pfeils P₂, wodurch das Mittelstück 34 gegen die Leitung 20 drückt und diese klemmt, wodurch ein Durchfluss von Medium im Wesentlichen verhindert wird.

Wie insbesondere den Fig. 7 und 8 zu entnehmen ist, jedoch auch den Fig. 3 und 4, ist die Formgebung der Einlegevorrichtung über den Querschnitt hinweg unterschiedlich. Ein zu dem Patienten weisendes Ende 100 ist mit einem abgestuften Teil stück 102 versehen, wohingegen das andere Ende 101, das beispielsweise zu einem Infusionsbeutel hin zeigt, mit einem schrägen Absatz 103 versehen und ansonsten gerade ausgebildet ist.

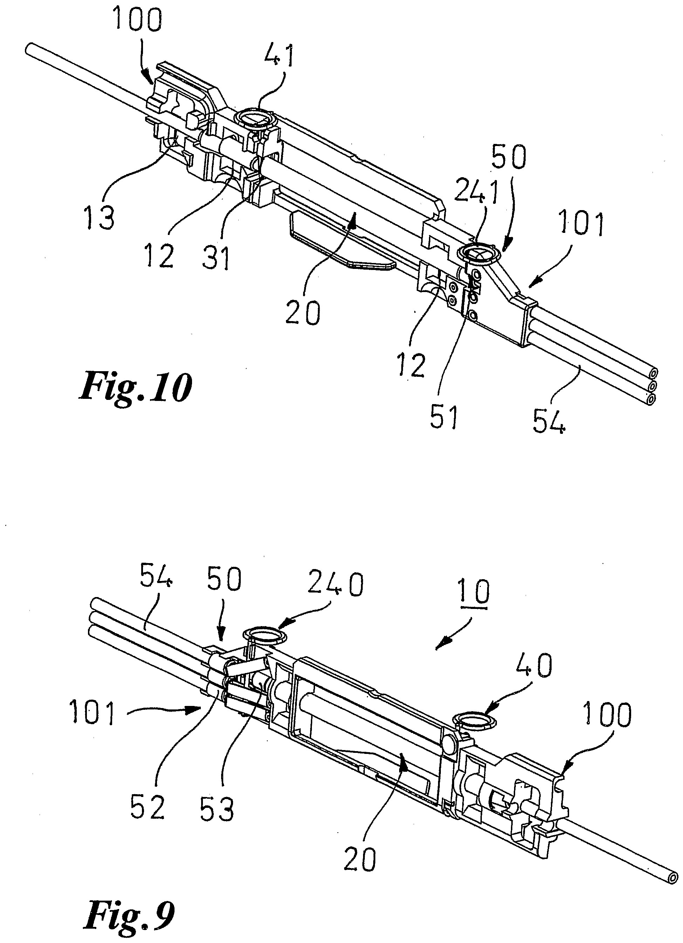

Eine andere Ausführungsform einer erfindungsgemäßen Einrichtung 10 ist in den Fig. 9 bis 13 in perspektivischer Ansicht dargestellt. Die Ausführungsform ist eine mehrkanalige Variante mit einer Mehrkanalumschalteinrichtung 50. Diese ist am Ende 101 der Einlegevorrichtung angefügt. Um einen Verpolungsschutz vorzusehen, also ein Anordnen der Mehrkanalumschalteinrichtung in falscher Richtung (180° gewendeter Richtung) zu vermeiden, werden Stößel 55 verwendet, die bei der Mehrkanalumschalteinrichtung 50 ansonsten zum Vermeiden des Durchtritts von Medium durch die gerade nicht verwendeten Leitungen verwendet werden, nach Art eines Ventils. Die Mehrkanalumschalteinrichtung 50 passt nur in der richtigen Anordnungsposition in die Pumpeinrichtung hinein. Da die äußere Formgebung der Einlegevorrichtung der ein- und mehrkanaligen Variante in den Figuren im Wesentlichen gleich ausgebildet ist, können die Stößel 55 zur Unterscheidung dieser beiden Varianten verwendet werden. Stößel sind bei der einkanaligen Variante nicht vorgesehen.

Die Mehrkanalumschalteinrichtung weist an ihrem Ende 101 drei Zuflussstutzen 52 auf. Zu der Einlegevorrichtung weisend ist ein Abflussstutzen 53 vorgesehen, wie dies noch besser Fig. 11 entnommen werden kann. Anstelle von drei Zuflussstutzen können auch nur zwei oder auch mehr als drei vorgesehen werden, insbesondere sind drei bis fünf vorgesehen. Die einzelnen Zuflussstutzen 52 können mit unterschiedlichen Infusionsbeuteln oder anderweitigen Medikamentenspendern etc. verbunden werden. Die Mehrkanalumschalteinrichtung 50 dient dazu, diese einzelnen Quellen, die über Leitungen 54 an die Zuflussstutzen 52 angeschlossen werden, mit dem Abflussstutzen 53 zu verbinden. Dies kann entweder sequentiell nacheinander oder aber auch durch Kombination mehrerer Zuflussstutzen erfolgen.

Die Mehrkanalumschalteinrichtung 50 weist neben den Stößeln 55 zum Klemmen einzelner Leitungen außerdem noch Federelemente 56 und diese in entsprechenden Öffnungen 57 der Einlegevorrichtung befestigende Niete 58 auf. Deren Anordnung kann besonders gut den Fig. 9 und 11 entnommen werden. Die Federelemente sind so angeordnet, dass sie mit den Stößeln zusammenwirken, wobei die jeweils zu klemmenden Schlauchabschnitte sich im Bereich der Zuflussstutzen 52 befinden. Hier greifen Enden 156 der Federelemente 56 an, wobei diese Enden abgewinkelt sind gegenüber der sonstigen Längserstreckung der Federelemente. Um entsprechend auch an der Stelle der Mehrfachumschalteinrichtung neben der Freiflussklemmeinrichtung bzw. den -einrichtungen einen Freifluss arretieren zu können, ist eine entsprechende Freiflussarretiereinrichtung 240 vorgesehen. Die Freiflussarretiereinrichtung 240 umfasst ebenfalls einen Griffabschnitt 241 sowie einen Arretierabschnitt 242. Die Freiflussarretiereinrichtung wird im Wesentlichen senkrecht durch eine Aussparung 243 in der Wandung der Einlegevorrichtung und unter den Federelementen 56 hindurch an der Einlegevorrichtung montiert. Dadurch, dass der Arretierabschnitt 242 zwischen der entsprechenden Wandung bzw. dem oder den Stößeln und den Federelementen angeordnet ist, werden die Federelemente von den Zuflussstutzen weggedrückt, so dass diese nicht abgeklemmt werden. Wird hingegen die Freiflussarretiereinrichtung 240 nach oben herausgezogen aus dem Bereich der Federelemente, können diese die entsprechenden Abschnitte der Zuflussstutzen 52 klemmen und damit den Freifluss von Medium verhindern.

Zum Verbinden des Abflussstutzens 53 mit der Leitung 21 ist zusätzlich ein Verbindungsstück 51 vorgesehen, das ähnlich dem Verbindungsstück 23 ausgebildet sein kann. Es kann hier jedoch auch direkt die Leitung 20 oder 21 in den Abflussstutzen 53 eingefügt werden. Die einzelnen Stößel 55 können mit den Federelementen 56 so zusammenwirken, dass jeweils das oder die Federelemente durch die Stößel nach außen weggedrückt werden, die den Zuflussstutzenabschnitt klemmen, durch den Medium strömen soll.

Zwischen den drei Zuflussstutzen 52 und dem Abflussstutzen 53 ist ein senkrecht angeordneter Verbindungskanal 59 angeordnet, der die jeweiligen Stutzen miteinander verbindet. Dieser wird so in der Einlegevorrichtung angeordnet, dass kein Medium ungewollt aus diesem Verbindungskanal austritt.

Wie bereits erwähnt, weist die einkanalige ebenso wie die mehrkanalige Variante der Einlegevorrichtung einen Verpolungsschutz auf, der auch hier in der unterschiedlichen Formgebung der beiden Enden 100 und 101 bzw. dem Vorsehen von Stößeln besteht. Die Kompaktheit der Einlegevorrichtung ist ebenso gegeben wie die dadurch mögliche einfache Handhabung. Die Leitungen von den Quellen und zum Ziel sind an die Einlegevorrichtung angeschlossen, also beispielsweise Infusionsbeutel und die Leitung zum Patienten bzw. ein Infusionsbesteck. Es wird somit eine vollständig ausgerüstete Einheit bereitgestellt, wobei die Einlegevorrichtung in eine Pumpvorrichtung eingefügt ist oder wird. Dadurch entfällt eine lange Vorbereitungszeit durch mühsames Einfädeln des Schlauches in die Einlegevorrichtung, wie dies im Stand der Technik insbesondere der EP 0 813 430 B1 erforderlich ist.

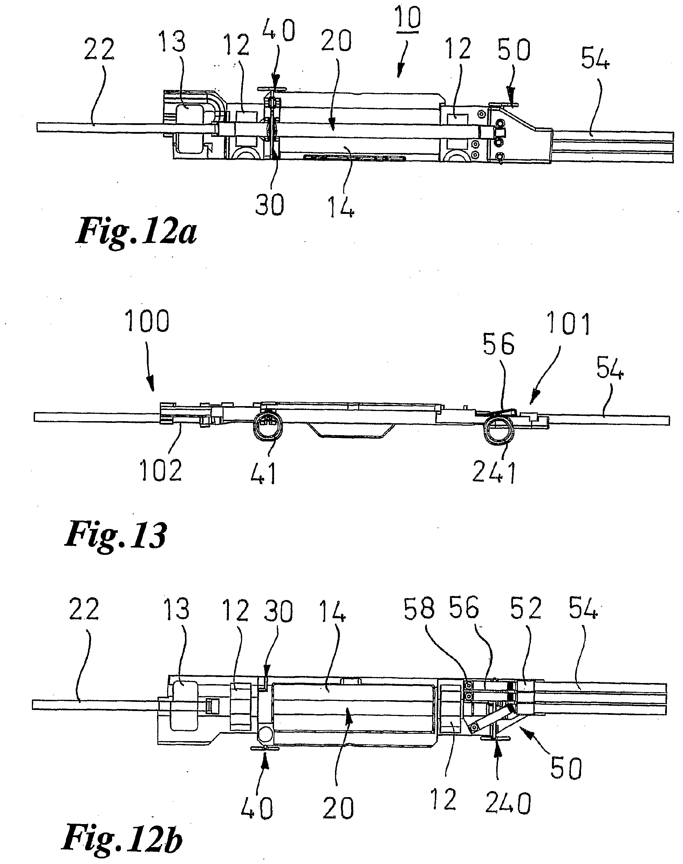

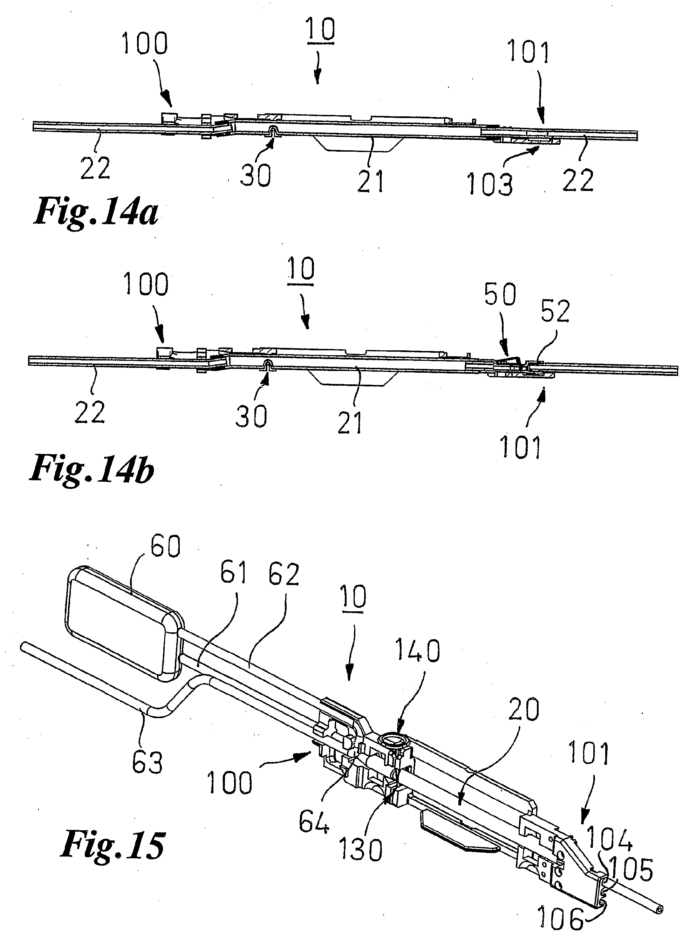

Wie insbesondere dem Vergleich in den Fig. 14a und 14b, jedoch auch den Fig. 8 und 13 zu entnehmen, ist es problemlos möglich, im Bereich des Endes 101 ein Sensormittel vorzusehen, das die unterschiedliche Formgebung der einkanaligen und mehrkanaligen Variante erkennt und entsprechend an eine nicht dargestellte Steuerungseinrichtung der Pumpvorrichtung meldet, so dass diese das entsprechende Programm starten kann. Das Sensormittel können z.B. die Stößel in Verbindung mit den Federelementen sein. Die Gesamtlänge der einkanaligen und mehrkanaligen Variante entspricht vorzugsweise einander, um diese beliebig gegeneinander austauschen zu können (aus Fig. 14 a und 14b ersichtlich). Es kann also in beiden Fällen die gleiche Pumpvorrichtung verwendet werden, so dass die Variabilität der Verwendung vorteilhaft erhöht wird. Ein freier Austausch einer ein- und mehrkanaligen Variante einer Einlegevorrichtung ist aus dem vorstehend genannten Stand der Technik nicht bekannt.

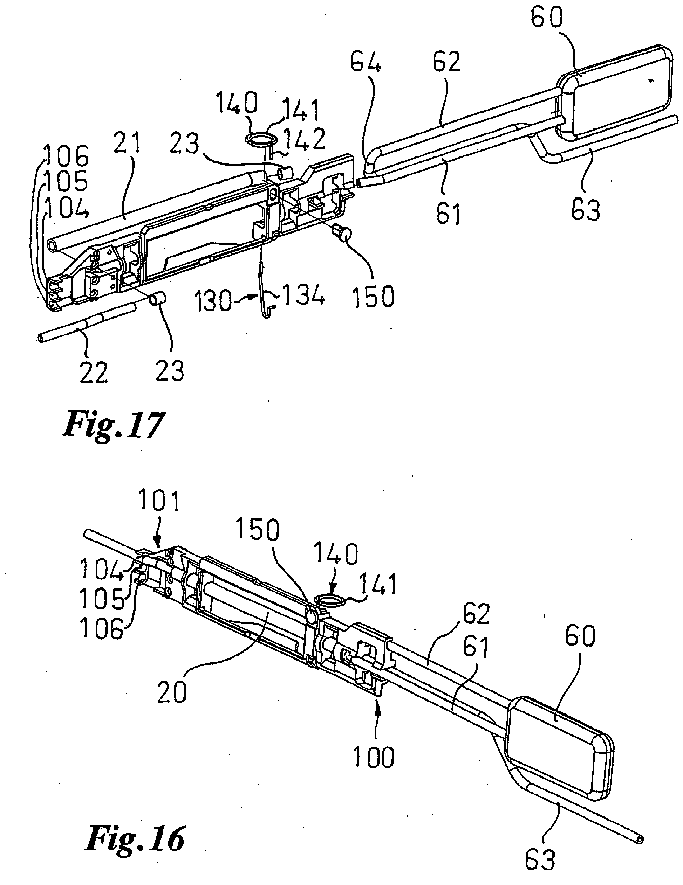

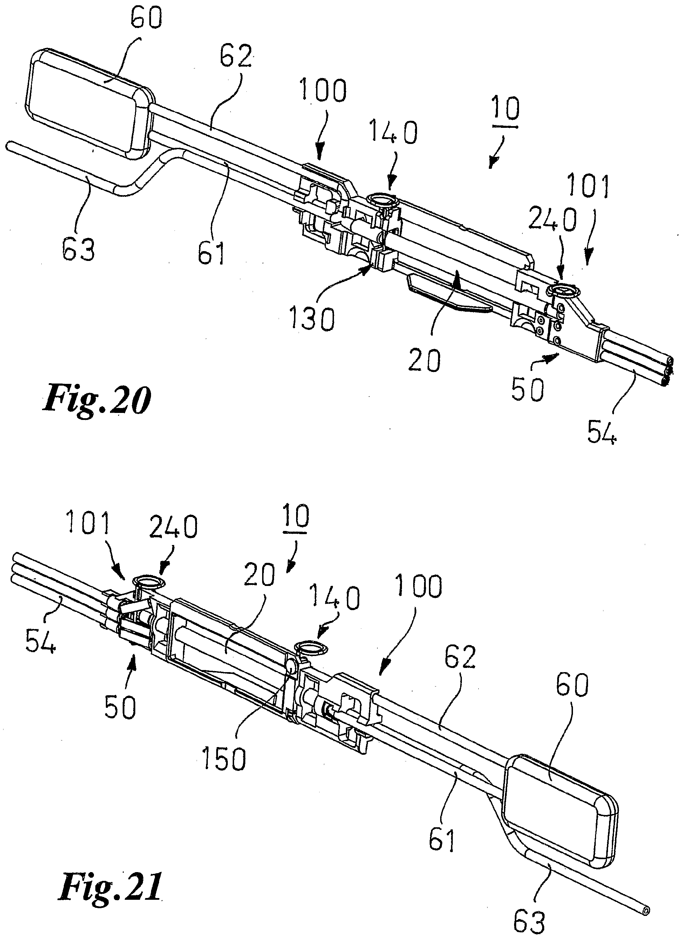

Die Figuren 15 bis 22 zeigen weitere Ausführungsformen einer erfindungsgemäßen einkanaligen und mehrkanaligen Einlegevorrichtung 10. Bei diesen Ausführungsformen ist insbesondere ein Filterelement 60 mit einer Zuleitung 61 und einer Ableitung 62 vorgesehen. Hierüber kann beim Entlüften des Schlauchsystems eine Filterung von in diesem enthaltener Luft vorgenommen werden. Über einen separat vorgesehenen Sensor, der in den Figuren 15 und 16 nicht dargestellt ist, kann der Luftgehalt innerhalb des Schlauchsystems gemessen werden, nachdem das Medium im Schlauchsystem aus der Einlegevorrichtung heraus, in das Filterelement 60 hinein geleitet, dort entlüftet und zur Einlegevorrichtung zurückgeleitet wurde. Wird zu viel Luft im Schlauchsystem festgestellt, kann ein Stoppen des Pumpvorgangs ausgelöst werden. Vorzugsweise ist das Filterelement am Ende 100, das zum Patienten führt, angeordnet. Die zum Patienten führende Leitung 63, die über einen Umlenkabschnitt 64 mit der Ableitung 62 verbunden ist, erstreckt sich in Fig. 15 bis 17 im Wesentlichen parallel zu der Zu- und Ableitung.

Das zum Infusionsbeutel oder einem ähnlichen Element weisende Ende 101 ist entsprechend der Mehrkanal-Ausführungsform ausgebildet. In den Aussparungen 104, 105, 106 ist jedoch nur ein Leitungsendstück 22 angeordnet. Gegenüber der Mehrkanal-Ausführungsform fehlen außerdem die Stößel und Federelemente, wie dem Vergleich mit den Fig. 19 bis 21 entnommen werden kann.

Die Freiflussklemmeinrichtung 130 sowie die Freiflussarretiereinrichtung 140 unterscheiden sich nicht von den Ausführungsformen der Freiflussklemmeinrichtung 30 und der Freiflussarretiereinrichtung 40 gemäß den vorstehend beschriebenen Figuren. Die Freiflussklemmeinrichtung 330 kann jedoch, wie in Fig. 23 gezeigt, nicht mehr durch eine Durchführöffnung und einen Schlitz an die Einlegevorrichtung angeklemmt sein, sondern die Wandung 16 der Einlegevorrichtung umgeben. In Fig. 23 ist sie mit ihrem Halteteilstück 335 auf der Außenseite 16a der Wandung 16 angelagert und weist auf der gegenüberliegenden Seite der Wandung 16 ein Klemmteilstück 333 mit einem ausbauchenden Abschnitt 334 auf. Dieses Klemmteilstück 333 mit dem ausbauchenden Abschnitt 334 ist auf das Halteteilstück 335 zurückgebogen und endet in einem Betätigungsteilstück 332. Dieses ist in einem Arretierstück 150 in einer darin vorgesehenen Öffnung 151 gehalten. Das Arretierstück weist eine weitere Öffnung 152 auf, in die ein Arretierabschnitt 142 der Freiflussarretiereinrichtung 140 eingefügt ist. Der Arretierabschnitt 142 ist im Wesentlichen rechtwinkelig zu einem Griffabschnitt 141 ausgebildet, der bevorzugt aufgrund des einfachen Angreifens ringförmig ist. Hierdurch ist besonders gut ein Angreifen an dem Griffabschnitt möglich. Die Form des Griffabschnitts 141 kann jedoch beliebig gewählt werden, solange ein Angreifen daran möglich ist.

Der Arretierabschnitt 142 stützt sich auf einem Stück der Wandung 16 neben einer Öffnung 16b der Wandung ab. In die Öffnung ragt das Arretierstück 150 hinein. Wird der Arretierabschnitt 142 aus der Öffnung 152 herausgezogen, erfolgt keine Abstützung des Arretierstücks 150 innerhalb der Öffnung 16b der Wandung 16 mehr, so dass die als federndes Element wirkende Freiflussarretiereinrichtung 140 das Arretierstück 150 durch die Öffnung 16b hindurchdrücken kann. Das Klemmteilstück 133 bewegt sich gegen die Wandung 16, so dass die Leitung 20 geklemmt wird. Im Bereich der Leitung 20 drückt die Freiflussklemmeinrichtung 130 mit ihrem ausbauchenden Abschnitt 134 gegen die Leitung 20. Aufgrund des Vorsehens einer der Leitungsform der Leitung 20 angepasst geformten Mulde 16c innerhalb der Wandung, wobei die Leitung in dieser Mulde liegt und die Formgebung des ausbauchenden Abschnitts 134 an die Formgebung der Mulde angepasst ist, kann ein Abquetschen der Leitung wiederum vorteilhaft vermieden werden.

Figur 18 zeigt die Einlegevorrichtung gemäß Figur 15 bis 17 in der Rückansicht, wobei hier beispielhaft auch Bemaßungsangaben vorgesehen sind. Die Länge der Einlegevorrichtung beträgt in dem hier dargestellten Fall z.B. 1 = 134 mm, die Breite B der Einlegevorrichtung z.B. 23,5 mm und die Breite des absetzten Abschnitts am Ende 100 z.B b = 19 mm bzw. an seinem schmalen Ende z.B. c = 12 mm. Dies sind lediglich beispielhafte Angaben, die die kleine Bauform der Einlegevorrichtung verdeutlichen sollen.

Die Figuren 19 bis 21 zeigen eine weitere Ausführungsform einer erfindungsgemäßen mehrkanaligen Einlegevorrichtung. Auch bei dieser ist wiederum ein Filterelement 60 mit Zu- und Ableitung 61, 62 vorgesehen. Anstelle der gezeigten drei können auch z.B. fünf Leitungen 54 vorgesehen werden. Es kann eine beliebige Anzahl von Leitungen 54 vorgesehen werden, die mit einzelnen Quellen, wie beispielsweise einem Medikamentenspender oder unterschiedlichen Infusionsbeuteln etc. verbunden werden können. Das Filterelement ist wiederum an dem zum Patienten weisenden Ende 100 angeordnet.

Auch in dieser Ausführungsform einer mehrkanaligen Einlegevorrichtung kann nach der Rückkehr des Mediums dem Filterelement 60 zur Einlegevorrichtung in dieser im Bereich des Umlenkabschnitts 64 ein Luftsensor 5 vorgesehen werden, der den Luftgehalt in der Leitung kontrolliert, wobei ggf. ein Abbruch des Pumpvorgangs bei zu hohem Luftgehalt ausgelöst wird.

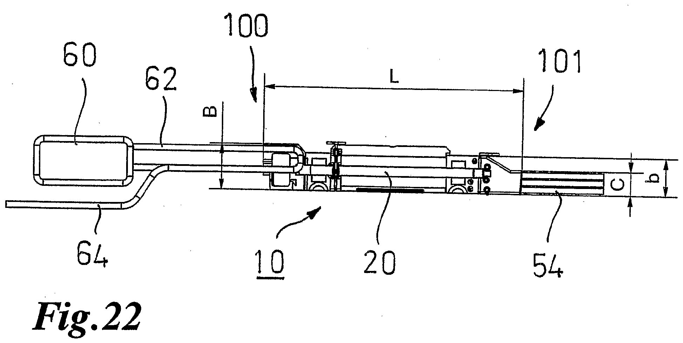

Figur 22 dient dem Verdeutlichen der geringen Baugröße der Einlegevorrichtung auch in der mehrkanaligen Form, die ebenfalls z.B. eine Länge von 1 = 134 mm, eine Breite B von z.B. 23,5 mm, eine Breite b im Endbereich des Endes 100 von z.B. 19 mm sowie eine Breite c am Ende der Einlegevorrichtung von z.B. c = 12 mm aufweist. Hierdurch ist ein freier Austausch der ein- und mehrkanaligen Ausführungsform innerhalb einer Pumpvorrichtung möglich. Wie bereits zu Figur 18 erwähnt, können die Abmessungen jedoch auch beliebig anders ausgewählt werden. Insbesondere können sie auch noch geringer oder aber größer sein, je nach anwenderbezogenem Anwendungsfall.

Die Einlegevorrichtung besteht vorzugsweise aus einem Kunststoff, wohingegen das Element aus federelastischem Material bevorzugt aus einem Metall, insbesondere Federstahl besteht. Es kann jedoch auch ein beliebig anderes Rückstelleigenschaften aufweisendes Material verwendet werden. Die Einlegevorrichtung selbst besteht insbesondere aus einem stabilen Material, das ein für eine Leitung bzw. einen Schlauch verletzungsfreies Einlegen und Führen derselben ermöglicht. Es kann somit auch ein beliebiges anderes Material anstelle von Kunststoff verwendet werden, das diese Eigenschaft aufweist.

Neben den im Vorstehenden beschriebenen und in den Figuren dargestellten Ausführungsbeispielen können noch zahlreiche weitere gebildet werden, bei denen jeweils die Einlegevorrichtung zum Einlegen einer Leitung ausgebildet und mit einer Freiflussklemmeinrichtung versehen ist, die ein federndes Element aufweist, das so an der Einlegevorrichtung angebracht werden kann, dass zwischen diesem und der Einlegevorrichtung ein Raum verbleibt, in den die ein- oder mehrteilige Leitung eingefügt werden kann bzw. nach Einfügen der Leitung das federnde Element die Leitung zumindest teilweise umgebend an der Einlegevorrichtung befestigt wird. Anstelle einer C-Form ist beispielsweise jede andere beliebige Form des federnden Elementes möglich, die es gestattet, das Element so an der Einlegevorrichtung zu befestigen, dass es von außerhalb der Einlegevorrichtung betätigt werden kann und ohne Betätigung ein Klemmen der Leitung innerhalb der Einlegevorrichtung zulässt bzw. ausübt.

### Bezugszeichenliste

- 1: Gehäuse
- 2: peristaltische Antriebseinrichtung
- 3: Verschlussklappe
- 4: Scharniergelenk
- 5: Luftsensor
- 6: Drucksensor
- 7: Drucksensor
- 10: Einlegevorrichtung
- 11: Spalt
- 12: Ausnehmung für Drucksensor
- 13: Aussparung für Luftsensor
- 14: Aussparung für Antriebseinrichtung
- 16: Wandung
- 16a: Außenseite
- 16b: Öffnung
- 16c: Mulde
- 17: Durchführöffnung bzw. Schlitz
- 18: Schlitz
- 19: Öffnung
- 20: Leitung
- 21: mittleres Leitungsstück
- 22: Leitungsendstück
- 23: Verbindungsstück
- 30: Freiflussklemmeinrichtung
- 31: Element aus federelastischem Material
- 33: Betätigungsteilstück
- 34: Mittelstück
- 35: Halteteil stück
- 40: Freiflussarretiereinrichtung
- 41: Griffelement
- 42: Arretierelement
- 43: auskragendes Ende
- 45: umlaufende Auskragung
- 50: Mehrkanalumschalteinrichtung
- 51: Verbindungsstück
- 52: Zuflussstutzen
- 53: Abflussstutzen
- 54: Leitung
- 55: Stößel
- 56: Federelement
- 57: Öffnung
- 58: Niet
- 59: Verbindungskanal
- 60: Filterelement
- 61: Zuleitung
- 62: Ableitung
- 63: zum Patienten führende Leitung
- 64: Umlenkabschnitt
- 100: Ende zum Patienten
- 101: Ende zum Infusionsbeutel
- 102: abgestuftes Teilstück
- 103: schräger Absatz
- 104: Aussparung
- 105: Aussparung
- 106: Aussparung
- 116: Ausnehmung
- 130: Freiflussklemmeinrichtung
- 132: Betätigungsteil stück
- 133: Klemmteilstück
- 134: ausbauchender Abschnitt
- 135: Halteteilstück
- 140: Freiflussarretiereinrichtung
- 141: Griffabschnitt
- 142: Arretierabschnitt
- 150: Arretierstück
- 151: Öffnung
- 152: Öffnung
- 153: Endflansch
- 156: abgewinkeltes Ende
- 240: Freiflussarretiereinrichtung
- 241: Griffabschnitt
- 242: Arretierabschnitt
- 243: Aussparung
- 330: Freiflussklemmeinrichtung
- 332: Betätigungsteilstück
- 333: Klemmteilstück
- 334: ausbauchender Abschnitt
- 335: Halteteilstück
- 1: Länge der Einlegevorrichtung
- B: Breite der Einlegevorrichtung
- b: Breite am Endabschnitt beim Ende 100
- C: Breite am Ende 100
- P₁: Pfeil
- P₂: Pfeil

## Patentansprüche

1. Einlegevorrichtung (10) für eine Pumpvorrichtung, insbesondere eine Infusionspumpe, mit zumindest einer in einer ersten Aussparung oder Ausnehmung (11) eingelegten Leitung (20) und mit zumindest einer Freiflussklemmeinrichtung (30, 130),
wobei die zumindest eine Freiflussklemmeinrichtung (30, 130) ein einteiliges Element (31) aus federelastischem Material mit einem an der Einlegevorrichtung angreifenden Halteteilstück (35, 135), einem auf die Leitung (20) einwirkenden Mittelstück (34, 134) und einem Betätigungsteilstück (33, 132) aufweist, wobei das Element (31) so angeordnet ist, dass zwischen diesem und einer Wandung (16) der Einlegevorrichtung (10) eine Durchführung (17) für die Leitung (20) verbleibt und die eingelegte Leitung (20) zwischen dem Mittelstück (34, 134) und der Wandung (16) klemmbar und bei einem Betätigen des Betätigungsteilstücks (33, 132) lösbar ist,
wobei die Einlegevorrichtung (10) im Bereich der Leitungsdurchführung (11) zumindest eine zweite Aussparung (12, 13) zum Einfügen einer Sensoreinrichtung (5, 6, 7) der Pumpvorrichtung an die Leitung (20) und zumindest eine dritte Aussparung (14) zum Eingreifen einer Antriebseinrichtung (2) der Pumpvorrichtung an die Leitung (20) aufweist,
wobei die Freiflussklemmeinrichtung (30, 130) zwischen der Aussparung (14) für die Antriebseinrichtung (2) und der Aussparung (12, 13) für die Sensoreinrichtung (5, 6, 7) angeordnet ist,
und wobei die Leitung (20) durch die in Längsrichtung angeordnete erste Aussparung oder Ausnehmung (11) sowie die zweite Aussparung (12, 13) und die dritte Aussparung (14) hindurchläuft.

2. Einlegevorrichtung (10) nach Anspruch 1,
**dadurch gekennzeichnet, dass**
das Betätigungsteilstück (33) so in der Einlegevorrichtung (10) und/oder in einem weiteren Element (150) angeordnet oder anordbar ist, dass es und/oder dieses Element (150) von außerhalb der Einlegevorrichtung zugänglich ist, insbesondere für das Einwirken zumindest eines Teils einer Verschlussklappe (3) der Pumpvorrichtung zum Lösen und Auslösen einer Klemmung.

3. Einlegevorrichtung (10) nach Anspruch 1 oder 2,
**dadurch gekennzeichnet, dass**
die zumindest eine Freiflussklemmeinrichtung (30, 130) durch zumindest eine mit dem Betätigungsteilstück (33) zusammenwirkende oder direkt oder indirekt auf dieses einwirkende Freiflussarretiereinrichtung (40, 140) in einer Freiflussposition arretierbar ist.

4. Einlegevorrichtung (10) nach Anspruch 3,
**dadurch gekennzeichnet, dass**
die zumindest eine Freiflussarretiereinrichtung (40, 140) ein Griffelement (41, 141) und ein Arretierelement (42, 142), insbesondere ein zwischen das Element (31) aus federelastischem Material und die Einlegevorrichtung (10) einfügbares Arretierelement (42) aufweist.

5. Einlegevorrichtung (10) nach Anspruch 4,
**dadurch gekennzeichnet, dass**
das Arretierelement (42, 142) stiftförmig ausgebildet ist, insbesondere ein auskragendes Ende (43) aufweist.

6. Einlegevorrichtung (10) nach einem der vorstehenden Ansprüche,
**dadurch gekennzeichnet, dass**
das Halteteilstück (35) der Freiflussklemmeinrichtung (30) in eine Aussparung, Ausnehmung, Nut, insbesondere einen Schlitz (18), der Einlegevorrichtung (10) so weit eingreift, dass zumindest das Betätigungsteilstück (33) sich klemmend an der Einlegevorrichtung (10) anlegt oder in ein Arretierstück (150) eingreift.

7. Einlegevorrichtung (10) nach einem der vorstehenden Ansprüche,
**dadurch gekennzeichnet, dass**
die Sensoreinrichtung (5, 6, 7) ein Drucksensor (6, 7) ist.

8. Einlegevorrichtung (10) nach einem der vorstehenden Ansprüche,
**dadurch gekennzeichnet, dass**
eine Verpolungsschutzeinrichtung zum Kennzeichnen der Einlegerichtung der Einlegevorrichtung (10) in der Pumpvorrichtung vorgesehen ist.

9. Einlegevorrichtung (10) nach einem der vorstehenden Ansprüche,
**dadurch gekennzeichnet, dass**
die Einlegevorrichtung (10) einkanalig und/oder mehrkanalig ausgebildet ist, insbesondere eine mit einer einkanaligen Einlegevorrichtung verbindbare oder verbundene Mehrkanalumschalteinrichtung (50) vorgesehen ist.

10. Einlegevorrichtung (10) nach Anspruch 9,
**dadurch gekennzeichnet, dass**
die Mehrkanalumschalteinrichtung (50) zumindest ein Verbindungselement (52, 53, 59, 56), insbesondere einen Verbindungsabschnitt nach Art eines Ventilkörpers und die Einlegevorrichtung (10) Nuten, Öffnungen oder dergleichen Einrichtungen zum Einfügen des Verbindungselementes aufweist.

11. Einlegevorrichtung (10) nach einem der vorstehenden Ansprüche,
**dadurch gekennzeichnet, dass**
sensorerfassbare Teilbereiche der Einlegevorrichtung (10) zum Unterscheiden einer ein- von einer mehrkanaligen Einlegevorrichtung (10) unterschiedlich ausgeführt, insbesondere mit unterschiedlichen Dicken und/oder unterschiedlich angeordneten Absätzen (103) und/oder Elementen versehen sind.

12. Einlegevorrichtung (10) nach einem der vorstehenden Ansprüche,
**dadurch gekennzeichnet, dass**
ein Filterelement (60) mit der Leitung (20) verbindbar und so angeordnet ist, dass in der Leitung (20) befindliches Medium zum Entlüften durch das Filterelement (60) hindurch und von diesem zurück an einer Sensoreinrichtung (5) vorbei leitbar ist.

13. Einlegevorrichtung nach einem der Ansprüche 9 bis 12,
**dadurch gekennzeichnet, dass**
eine weitere Freiflussarretiereinrichtung (240) im Bereich der Mehrkanalumschalteinrichtung (50) vorgesehen ist, die mit zumindest einer Freiflussklemmeinrichtung (55, 56) zusammenwirkt.

## Claims

1. Insert device (10) for a pump device, especially an infusion pump, with at least one line (20) inserted into a first relief or recess (11) and with at least one anti-free flow clamping device (30, 130),
wherein the at least one anti-free flow clamping device (30, 130) has a one-part element (31) produced from a spring elastic material with retaining section (35, 135) acting upon the insert device, a center piece (34, 134) acting upon the line (20) and an actuating section (33, 132), wherein the element (31) is disposed in such a manner that it leaves a passage (17) for the line (20) between the element and a wall (16) of the insert device (10) and the inserted line is clampable between the center piece (34, 134) and the wall (16) and releasable upon actuating the actuating section (33, 132),
wherein the insert device (10) has in the area of the fine passage (11) at least a second recess (12, 13) for inserting a sensor device (5, 6, 7) of the pump device onto the line (20) and a third recess (14) for engaging of a drive equipment (2) of the pump device onto the line (20),
wherein the anti-free flow clamping device (30, 130) is located between recess (14) for the drive equipment (2) and the recess (12, 13) for the sensor device(5, 6, 7),
and wherein the line (20) runs through in a longitudinal direction arranged first relief or recess (11) as well as second recess (12, 13) and third recess (14).

2. Insert device (10) according to claim 1,
**characterized in that**
the actuating section (33) is arranged or arrangeable in such a way in the insert device (10) and/or in a further element (150), that it is accessible from outside of the insert device and/or the element (150), particularly for acting of at least a part of a cover flap (3) of the pump device for releasing and triggering of a clamping.

3. Insert device (10) according to claim 1 or 2,
**characterized in that**
the at least one anti-free flow clamping device (30, 130) is lockable in a free flow position by at least one free flow locking device (40, 140) cooperating with the actuating section (33) or directly or indirectly acting thereon.

4. Insert device (10) according to claim 3,
**characterized in that**
the at least one free flow locking device (40, 140) has a handle element (41, 141) and a locking element (42, 142), particularly a locking element (42) insertable between the element (31) of spring elastic material and the insert device (10).

5. Insert device (10) according to claim 4,
**characterized in that**
the locking element (42, 142) is pencil-shaped, particularly with a protruding end (43).

6. Insert device (10) according to one of the proceeding claims,
**characterized in that**
the retaining section (35) of the anti-free flow clamping device (30) engages so far into a relief, recess, notch, particularly a slit (18), of the insert device (10), that at least the actuating section (33) rest in a clamping way on the insert device (10) or engages into a locking part (150).

7. Insert device (10) according to one of the proceeding claims,
**characterized in that**
the sensor device (5, 6, 7) is a pressure sensor (6, 7).

8. Insert device (10) according to one of the proceeding claims,
**characterized in that**
a reverse polarity protection device is provided for identifying the insertion polarity of the insert device (10) into the pump device.

9. Insert device (10) according to one of the proceeding claims,
**characterized in that**
the insert device (10) is designed one-channeled and/or multi-channeled, particularly provided a with a one-channeled insert device connectable or connected multi-channel switch device (50).

10. Insert device (10) according to claim 9,
**characterized in that**
the multi-channel switch device (50) has at least one connecting element (52, 53, 59, 56), particularly a connecting section according to a valve body and the insert device (10) has nuts, openings or such the like for inserting the connecting elements.

11. Insert device (10) according to one of the proceeding claims,
**characterized in that**
sensor detectable sections of the insert device (10) are designed differently for distinguishing of a one- from a multi-channeled insert device (10), particularly provided with different thicknesses and/or differently arranged sections (103) and/or elements.

12. Insert device (10) according to one of the proceeding claims,
**characterized in that**
a filter element (60) is connectable with the line (20) an arranged in such a way, that the media inside the line (20) is guidable through the filter element (60) for venting purposes and back from it to past a sensor device (5).

13. Insert device (10) according to one of claims 9 to 12,
**characterized in that**
a further free flow locking device is provided in the area of the multi-channel switch device (50), that cooperates with at least one anti-free flow clamping device (55, 56).

## Revendications

1. Dispositif d'insertion (10) pour un dispositif de pompage, notamment une pompe de perfusion, comprenant au moins une conduite (20) disposée dans un premier évidement ou une première cavité (11) et comprenant au moins un dispositif de blocage de flux libre (30, 130),
l'au moins un dispositif de blocage de flux libre (30, 130) comprenant un élément (31) monobloc en un matériau élastique, qui comprend une partie de retenue (35, 135) s'engageant de manière adjacente au dispositif d'insertion, une partie intermédiaire (34, 134) agissant sur la conduite (20) et une partie d'actionnement (33, 132), l'élément (31) étant disposé de sorte qu'il reste un passage (17) pour la conduite (20) entre celui-ci et une paroi (16) du dispositif d'insertion (10) et la conduite (20) insérée pouvant être serrée entre la partie intermédiaire (34, 134) et la paroi (16) et pouvant être desserrée par un actionnement de la partie d'actionnement (33, 132),
le dispositif d'insertion (10) comprenant au moins un deuxième évidement (12, 13) au niveau du passage de conduite (11) pour introduire un dispositif capteur (5, 6, 7) du dispositif de pompage de manière adjacente à la conduite (20) et au moins un troisième évidement (14) pour faire un dispositif d'entraînement (2) du dispositif de pompage s'engager auprès de la conduite (20),
le dispositif de blocage de flux libre (30, 130) étant disposé entre l'évidemment (14) du dispositif d'entraînement (2) et l'évidement (12, 13) du dispositif capteur (5, 6, 7),
et la conduite (20) passant par, dans la direction longitudinale, le premier évidement ou la première cavité (11) ainsi que par le deuxième évidement (12, 13) et par le troisième évidement (14).

2. Dispositif d'insertion (10) selon la revendication 1,
**caractérisé en ce que**
la partie d'actionnement (33) est disposée ou peut être disposée dans le dispositif d'insertion (10) et/ou dans un autre élément (150) de sorte que celle-ci et/ou cet élément (150) est accessible de l'extérieur du dispositif d'insertion, notamment pour l'action d'au moins une partie d'un clapet de fermeture (3) du dispositif de pompage pour desserrer et provoquer un blocage.

3. Dispositif d'insertion (10) selon la revendication 1 ou la revendication 2,
**caractérisé en ce qu'**
au moins un dispositif de blocage de flux libre (30, 130) peut être bloqué dans une position de flux libre par moyen d'un dispositif de verrouillage de position de flux libre (40, 140), qui coopère avec la partie d'actionnement (33) ou qui agit directement ou indirectement sur celle-ci.

4. Dispositif d'insertion (10) selon la revendication 3,
**caractérisé en ce que**
l'au moins un dispositif de verrouillage de position de flux libre (40, 140) comprend un élément de saisie (41, 141) et un élément d'arrêt (42, 142), notamment un élément d'arrêt (42), qui peut être inséré entre l'élément (31) en matériau élastique et le dispositif d'insertion (10).

5. Dispositif d'insertion (10) selon la revendication 4,
**caractérisé en ce que**
l'élément d'arrêt (42, 142) est conçu en forme de broche et comprend notamment une extrémité surplombant (43).

6. Dispositif d'insertion (10) selon l'une des revendications précédentes,
**caractérisé en ce que**
la partie de retenue (35) du dispositif de blocage de flux libre (30) s'engage dans un évidement, une cavité, une rainure, notamment une fente (18) du dispositif d'insertion (10) jusqu'à un tel point qu'au moins la partie d'actionnement (33) vienne s'appuyer de manière serrante contre le dispositif d'insertion (10) ou vienne en prise avec une pièce d'arrêt (150).

7. Dispositif d'insertion (10) selon l'une des revendications précédentes,
**caractérisé en ce que**
le dispositif capteur (5, 6, 7) est un capteur de pression (6, 7).

8. Dispositif d'insertion (10) selon l'une des revendications précédentes,
**caractérisé en ce qu'**
un dispositif de protection d'inversion de polarité pour marquer la direction d'insertion du dispositif d'insertion (10) est prévu dans le dispositif de pompage.

9. Dispositif d'insertion (10) selon l'une des revendications précédentes,
**caractérisé en ce que**
le dispositif d'insertion (10) est conçu avec un canal et/ou avec plusieurs canaux, et un dispositif de commutation multicanal (50) est notamment prévu, qui peut être relié ou qui est relié à un dispositif d'insertion à un canal.

10. Dispositif d'insertion (10) selon la revendication 9,
**caractérisé en ce que**
le dispositif de commutation multicanal (50) comprend au moins un élément de liaison (52, 53, 59, 56), notamment un section de liaison du type d'un corps de vanne, et le dispositif d'insertion (10) comprend des rainures, des ouvertures ou des dispositifs similaires pour insérer l'élément de liaison.

11. Dispositif d'insertion (10) selon l'une des revendications précédentes,
**caractérisé en ce que**
des sections susceptibles d'être détectées par un capteur du dispositif d'insertion (10) sont conçues de manière différente et sont notamment munies d'épaisseurs différentes et/ou d'épaulements (103) et/ou d'éléments disposés de manière différente pour distinguer un dispositif d'insertion (10) à un canal d'un dispositif d'insertion (10) à plusieurs canaux.

12. Dispositif d'insertion (10) selon l'une des revendications précédentes,
**caractérisé en ce qu'**
un élément de filtre (60) peut être relié à la conduite (20) et est disposé de sorte que du médium, qui se trouve dans la conduite, peut être guidé à travers l'élément de filtre (60) pour le dégazage et, en retour de celui-ci, peut être passé le long d'un dispositif capteur (5).

13. Dispositif d'insertion selon l'une des revendications 9 à 12,
**caractérisé en ce qu'**
un autre dispositif de verrouillage de position de flux libre (240) est prévu au niveau du dispositif de commutation multicanal (50), lequel coopère au moins avec un dispositif de blocage de flux libre (55, 56).
